(19) Europäisches Patentamt
European Patent Office
Office européen des brevets

(11) **EP 4 708 300 A1**

(12) **EUROPEAN PATENT APPLICATION**
published in accordance with Art. 153(4) EPC

(43) Date of publication:
**11.03.2026 Bulletin 2026/11**

(21) Application number: **24815270.4**

(22) Date of filing: **20.05.2024**

(51) International Patent Classification (IPC):
**G16B 15/00** (2019.01)   **G06F 16/332** (2025.01)
**G16B 20/00** (2019.01)

(52) Cooperative Patent Classification (CPC):
**G06F 16/332; G16B 15/00; G16B 20/00**

(86) International application number:
**PCT/JP2024/018489**

(87) International publication number:
**WO 2024/247786 (05.12.2024 Gazette 2024/49)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **26.05.2023 JP 2023086595**

(71) Applicants:
• **ADVANCED ACCELERATION SYSTEMS CO., LTD.**
**Kanagawa 236-0021 (JP)**
• **Kabushiki Kaisha Dnaform**
**Kanagawa 230-0051 (JP)**

(72) Inventor: **MAKINO, Junichiro**
**Yokohama-shi, Kanagawa 236-0021 (JP)**

(74) Representative: **TBK**
**Bavariaring 4-6**
**80336 München (DE)**

(54) **COMPUTER PROGRAM FOR INDEX CREATION, COMPUTER PROGRAM FOR MAPPING, COMPUTER DEVICE FOR INDEX CREATION, COMPUTER DEVICE FOR MAPPING, AND DATA STRUCTURE OF LAYERED INDEX**

(57)   The present invention is an index generation computer program for implementing the generation of an index to be used in identifying a partial character string of a reference character string based on a query character string. The index generation computer program includes the step of setting a number of levels from an uppermost level to a lower level in a suffix tree, which is obtained by hierarchically structuring a suffix array of the reference character string, to a predetermined value and thereby omitting hierarchical data at levels lower than the predetermined value. With this configuration, analysis of given query data using the reference data can be performed at high speed and/or efficiently.

Fig. 7

EP 4 708 300 A1

**Description**

Technical Field

The present invention relates to an index generation computer program and a mapping computer program suitable for genome analysis, apparatuses that execute these programs, and a data structure of a hierarchical index used by these programs.

Background Art

The human genome is a set of genetic information possessed by humans, and the substance that carries this information is DNA (deoxyribonucleic acid), which consists of approximately 3 billion pairs of bases linked together. The bases are adenine (A), guanine (G), cytosine (C), and thymine (T). In other words, human genetic information is determined by the arrangement (sequence) of these bases.

The human genome is read using an apparatus called sequencer. The sequencer reads the human genome sample, fragments it to a predetermined upper limit value (approximately several hundreds of base pairs), amplifies the fragments, and outputs a huge data array consisting of hundreds of millions of genetic information data fragments. Existing sequencers can read out one person's human genome in about one hour.

The separate data fragments read out by the sequencer are reconstructed into an original-length human genome sequence for analysis through comparison with a human genome reference sequence defined as a standard human genome sequence. For example, the position of each data fragment is determined (mapping) through comparison with the human genome reference sequence. After the completion of the mapping, analysis is performed to identify what types of mutations are present. Since the data fragments read from the sequencer typically contain errors, statistical processing is performed using redundant data of, e.g., 30 times a single human genome sequence per sample for the sake of error reduction. Human genome sequence analysis thus requires an enormous amount of computation, and high-performance computers such as supercomputers, cluster computers, and FPGA (Field-Programmable Gate Array) based computers are typically used.

The data fragments are mapped using program tools such as BWA (Burrows-Wheeler Aligner), for example. BWA includes three algorithms: BWA-backtrack, BWA-SW, and BWA-MEM. Of these, BWA-MEM is widely used as a fast algorithm for handling indels (insertions and deletions). BWA-MEM is an algorithm that generates an index using a suffix array for those portions of query character strings based on the read data fragments that occur repeatedly in the human genome reference sequence, and performs mapping (Heng Li, "Aligning sequence reads, clone sequences and assembly contigs with BWA-MEM", May 26, 2013, arXiv: 1303.3997 (q-bio.GN)).

The mapped data fragments are collated with partial sequences at the same positions in the human genome reference sequence, and analysis is performed to identify what types of mutations are present. The partial sequences of the human genome reference sequence and the data fragments are collated using an alignment algorithm, for example. The alignment algorithm identifies approximate character strings by dynamic programming while calculating the degree of variation (degree of similarity) for each element of an array called alignment table.

Summary of Invention

Technical Problem

The foregoing conventional BWA-MEM is widely used as an algorithm capable of high-speed mapping while allowing gaps, but has had the problem of requiring large amounts of memory resources because the size of the index generated from the reference sequence is extremely large. For example, in the case of huma genome analysis, the data volume of the index for the human genome reference sequence is enormous. Such an index is unable to be fully accommodated in a cache memory capable of faster access, and needs to be stored in external memory (main memory). As a result, there has been a problem that the external memory needs to be accessed a number of times corresponding to the number of characters (i.e., number of bases) in the query character string for traversal, and the processing speed of the processor is rate-limited by the memory access time.

More specifically, to identify the occurrence positions of a group of query character strings read by the sequencer in a fragmented state within the human genome reference sequence, BWA-MEM performs random access to the main memory a number of times corresponding to the total number of characters in the query character string group. In current computers, the latency of a single random access to the main memory (time from when access occurs to when data is actually obtained) is approximately 1 $\mu$sec (0.000001 sec) per bank. In the case of analyzing the human genome with 3 billion base pairs at a redundancy of 30, the total access time T for the total number of characters is:

$$T = 3,000,000,000 \times 0.000001 \times 30 = 90,000 \text{ (sec)}.$$

In other words, in human genome analysis, random access to the main memory alone takes approximately 90,000 seconds (approximately 25 hours). Even with the use of computers equipped with high-performance processors, there has thus been a limit to reducing the mapping time, with the memory access time being the constraining factor.

One of the objects of the present invention is to provide a technique for generating a hierarchical index based on a reference character string, suitable for mapping processing. Another object of the present invention is to provide a technique for performing mapping processing at high speed and/or efficiently that identifies the position where a data fragment (query character string) read by, e.g., a sequencer or the like occurs in the reference character string.

Solution to Problem

The present invention for solving the foregoing problem includes the following matters specifying the invention or technical features.

That is, the present invention related to the foregoing problem is an index generation computer program for causing a computing device to generate an index to be used in identifying a partial character string of a reference character string based on a query character string, the index generation computer program including the step of setting a number of levels from an uppermost level to a lower level in a suffix tree, which is obtained by hierarchically structuring a suffix array of the reference character string, to a predetermined value and thereby omitting hierarchical data at levels lower than the predetermined value.

Concerning the index generation computer program, the predetermined value of the number of levels may be set to 1000 or less.

Concerning the index generation computer program, the predetermined value of the number of levels may be set to be greater than or equal to a number of characters in the query character string.

Concerning the index generation computer program, the step of generating a supertree including a level higher than an intermediate level and a plurality of subtrees (hereinafter, referred to as a subtree group) including a level lower than the intermediate level and connected below the supertree by splitting the suffix tree at the intermediate level may be further included.

Concerning the index generation computer program, data volume of the subtrees may be set to a value capable of storage in a cache memory of the computing device.

Concerning the index generation computer program, data volume of the subtrees may be set to be less than or equal to less than or equal to 10 MB.

The present invention related to the foregoing problem is an index generation computer program for causing a computing device to generate an index to be used in identifying a partial character string of a reference character string based on a query character string, the index generation computer program including the step of generating a supertree including a level higher than an intermediate level and a plurality of subtrees (hereinafter, referred to as a subtree group) including a level lower than the intermediate level and connected below the supertree by splitting a suffix tree, which is obtained by hierarchically structuring a suffix array of the reference character string, at the intermediate level.

The present invention related to the foregoing problem is a mapping computer program for causing a computing device to implement a method for identifying a partial character string of a reference character string based on a query character string, the mapping computer program including the foregoing index generation computer program as a part, the method further including referring to the hierarchical index and mapping the query character string onto the reference character string to identify a partial character string of the reference character string that matches at least a part of the query character string.

The present invention related to the foregoing problem is a mapping computer program for causing a computing device to implement a method for identifying a partial character string of a reference character string based on a query character string, a hierarchical index generated based on the reference character string being split at an intermediate level and thereby including a supertree including a level higher than the intermediate level and a plurality of subtrees (hereinafter, referred to as a subtree group) including a level lower than the intermediate level and connected below the supertree, the method including referring to the hierarchical index and mapping the query character string onto the reference character string to identify a partial character string of the reference character string that matches at least a part of the query character string, the mapping of the query character string including a determination step of performing collation processing on the plurality of query character strings using the supertree and thereby determining which of the subtrees in the subtree group each of the query character strings belongs to, and an occurrence position determination step of performing collation processing using a specific one of the subtrees on the plurality of query character strings determined to belong to the specific one of the subtrees in the determination step and thereby determining occurrence positions of the query character strings relative to the reference character string.

Concerning the mapping computer program, the occurrence position determination step of the subtrees may be processed in parallel.

Concerning the mapping computer program, the determination step may be processed in parallel.

Concerning the mapping computer program, the subtrees may be loaded into a cache memory of the computing device in the occurrence position determination step.

Concerning the mapping computer program, the index generation computer program according to any one of the foregoing paragraphs may be included as a part.

The present invention related to the foregoing problem is an index generation computer apparatus including a computing device including a storage device, the index generation computer apparatus being configured to generate an index to be used in identifying a partial character string of a reference character string based on a query character string, the computing device including partial suffix tree generation means for reading the reference character string stored in the storage device, setting a number of levels from an uppermost level to a lower level in a suffix tree, which is obtained by hierarchically structuring a suffix array of the reference character string, to a predetermined value, and generating a partial suffix tree where hierarchical data at levels lower than the predetermined value is omitted.

The present invention related to the foregoing problem is an index generation computer apparatus including a computing device including a storage device, the index generation computer apparatus being configured to generate an index to be used in identifying a partial character string of a reference character string based on a query character string, the computing device including suffix tree splitting means for generating a supertree including an upper level and a plurality of subtrees (hereinafter, referred to as a subtree group) connected below the supertree by splitting a suffix tree, which is obtained by hierarchically structuring a suffix array of the reference character string stored in the storage device, at an intermediate level.

The present invention related to the foregoing problem is a mapping computer apparatus including a computing device including a storage device, the mapping computer apparatus being configured to identify a partial character string of a reference character string based on a query character string, the storage device storing a supertree including a level higher than an intermediate level and a plurality of subtrees (hereinafter, referred to as a subtree group) including a level lower than the intermediate level and connected below the supertree, the supertree and the subtree group being generated by splitting a hierarchical index, which is generated based on the reference character string, at the intermediate level, the computing device including mapping means for referring to the supertree and the subtrees and mapping the query character string onto the reference character string to identify a partial character string of the reference character string that matches at least a part of the query character string, the mapping means including determination means for referring to the supertree stored in the storage device, performing collation processing on the plurality of query character strings, and thereby determining which one of the subtrees in the subtree group each of the query character strings belongs to, and occurrence position determination means for referring to a specific one of the subtrees stored in the storage device, performing collation processing using a specific one of the subtrees on the plurality of query character strings determined to belong to the specific one of the subtrees by the determination means, and thereby determining occurrence positions of the query character strings relative to the reference character string.

The present invention related to the foregoing problem is a data structure of a hierarchical index of a reference character string to be used by a mapping computer program for causing a computing device to implement a method for identifying a partial character string of the reference character string based on a query character string, the data structure being a suffix tree obtained by hierarchically structuring a suffix array of the reference character string, a number of levels from an uppermost level to a lower level being set to a predetermined value, hierarchical data at levels lower than the predetermined value being omitted.

Concerning the data structure of the hierarchical index, the hierarchical index may include a data fragment of a supertree including a level higher than an intermediate level and data fragments of a plurality of subtrees including a level lower than the intermediate level and connected below the supertree by the suffix tree being split at the intermediate level.

The present invention related to the foregoing problem is a data structure of a hierarchical index of a reference character string to be used by a mapping computer program for causing a computing device to implement a method for identifying a partial character string of the reference character string based on a query character string, the hierarchical index being split at an intermediate level and thereby including a data fragment of a supertree including a level higher than the intermediate level and data fragments of a plurality of subtrees including a level lower than the intermediate level and connected below the supertree.

The present invention can also be implemented as a storage medium storing the foregoing computer programs. The present invention can also be implemented as an apparatus including hardware and/or firmware configured to perform the foregoing methods. If the index generation computer apparatuses, the mapping computer apparatuses, and the like are implemented through logical cooperation of a plurality of hardware components (or functional modules that implement specific functions), these apparatuses constitute index generation computer systems or mapping computer systems. If the index generation computer apparatuses, the mapping computer apparatuses, and the like are implemented by processor modules, these apparatuses constitute index generation processor modules or mapping processor modules. In other words, whether each apparatus or functional module is accommodated in a single housing, or whether each apparatus or functional module is implemented by a single functional component, does not particularly matter.

In this description and the like, means or a unit does not refer simply to physical means but also cover cases where the functions of the means or unit are implemented by software. The functions of single means or a single unit may be implemented by two or more physical means. The functions of two or more means or units may be implemented by single physical means.

Advantageous Effects of Invention

According to one of the present inventions, a hierarchical index that is based on a reference character string and suitable for mapping processing can be generated. With this hierarchical index, high-speed and/or efficient mapping processing can be implemented.

Moreover, according to the present invention, analysis using the human genome reference sequence based on data fragments read out by a sequencer can be performed at high speed and/or efficiently.

Other technical features, objects, operation, effects, and advantages of the present invention will become apparent from the following embodiment described with reference to the accompanying drawings. The effects described in this description are merely exemplary and not restrictive, and other effects may also be present.

Brief Description of Drawings

[Fig. 1] Fig. 1 is a block diagram showing an example of a schematic configuration of a computer system according to an embodiment of the present invention.
[Fig. 2] Fig. 2 is a flowchart for describing an example of an outline of approximate character string collation processing by the computer system.
[Fig. 3] Fig. 3 is a flowchart for describing an example of index generation processing by the computer system.
[Fig. 4] Fig. 4 is a diagram showing an example of cyclic sequences generated from a reference character string used in the computer system.
[Fig. 5] Fig. 5 is a diagram showing an example of a state where the cyclic sequences are sorted.
[Fig. 6] Fig. 6 is a diagram showing an example of a structure of a suffix tree generated based on the sorted cyclic sequences.
[Fig. 7] Fig. 7 is a diagram for describing a generation process of a partial suffix tree using the suffix tree.
[Fig. 8] Fig. 8 is a diagram showing an example of a structure of the partial suffix tree generated from the suffix tree.
[Fig. 9] Fig. 9(A) is a diagram for describing a splitting process of the partial suffix tree. Fig. 9(B) is a diagram showing an example of the split supertree.
[Fig. 10] Fig. 10 is a flowchart for describing an example of mapping processing by the computer system.
[Fig. 11] Fig. 11 is a diagram showing an example of query character strings used in the computer system.
[Fig. 12] Figs. 12(A) to 12(D) are diagrams for describing mapping processing by the computer system using subtrees.
[Fig. 13] Figs. 13(A) to 13(D) are diagrams for describing the mapping processing by the computer system using subtrees.
[Fig. 14] Figs. 14(A) to 14(D) are diagrams for describing the mapping processing by the computer system using subtrees.
[Fig. 15] Fig. 15 is a diagram showing a modification of the partial suffix tree.
[Fig. 16] Fig. 16 is a diagram for describing a modification in splitting the partial suffix tree.
[Fig. 17] Fig. 17 is a diagram showing an example of a hardware configuration of the computing device.
[Fig. 18] Fig. 18 is a diagram showing an example of a processor module in the hardware configuration of the computing device.
[Fig. 19] Fig. 19 is a block diagram showing an example of a functional configuration of the computing device.
[Fig. 20] Fig. 20 is a flowchart for describing a modification of the mapping processing by the computer system.
Description of Embodiments

Hereinafter, an embodiment of the present invention will be described with reference to the drawings. The following embodiment is just an example and not intended to exclude application of various modifications and techniques not explicitly described below. The present invention can be carried out with various modifications (for example, by combining embodiments) without departing from the gist thereof. In the following description of the drawings, the same or similar portions are denoted by the same or similar reference numerals. The drawings are schematic and do not necessarily match the actual dimensions, ratios, or the like. There may be portions where dimensional relationships or proportions vary between the drawings.

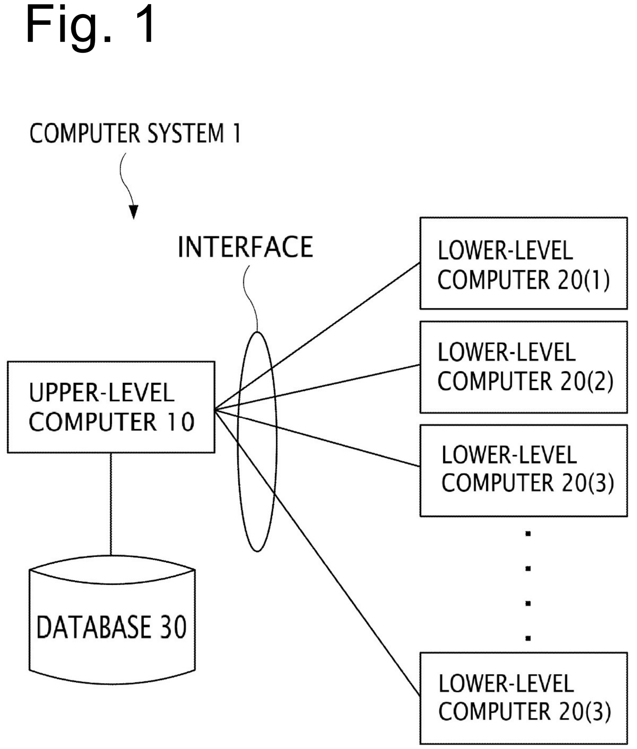

Fig. 1 is a block diagram showing an example of a schematic configuration of a computer system according to an embodiment of the present invention. As shown in the diagram, a computer system 1 includes an upper-level computer 10, a plurality of lower-level computers 20(1) to 20(n), and a database 30, for example. The upper-level computer 10 and the lower-level computers 20(1) to 20(n) are communicatively connected via a predetermined interface. In the present disclosure, the computer system 1 is configured as a centralized computer system where the upper-level computer 10 comprehensively controls the plurality of lower-level computers 20(1) to 20(n). However, this is not restrictive, and the computer system 1 may be configured as a distributed computer system, for example. In a distributed computer system, the computers can operate cooperatively through parallel distributed processing, whereas specific processing may be performed by one representative computer alone.

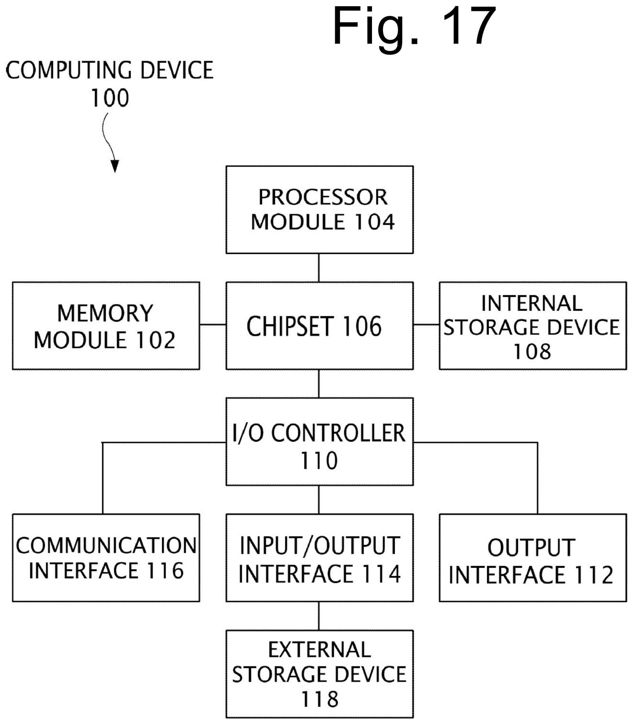

Fig. 17 shows a computing device 100, which is computational hardware common to the upper-level computer 10 and the lower-level computers 20. The computing device 100 includes a memory module 102 that serves as a main storage device such as a DRAM, a processor module 104 that serves as a central computer, a chipset 106 that serves as a bridge between the processor module 104 and external equipment, an internal storage device 108 such as an HDD and an SDD, an I/O controller 110, an output interface 112, an input/output interface 114, a communication interface 116, and an external storage device 118 such as an external HDD.

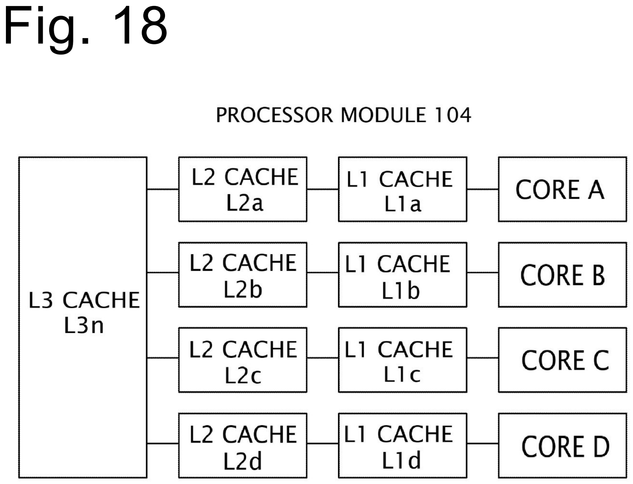

As shown in Fig. 18, the processor module 104 further includes a plurality of cores A, B, C, and D that perform computational processing independent of each other, first cache memories (L1 cache memories) L1a, L1b, L1c, and L1d and second cache memories (L2 cache memories) L2a, L2b, L2c, and L2d that are internal storage functions provided for the respective cores A, B, C, and D, and a third cache memory (L3 cache memory) L3n that is an internal storage function shared among all the cores A, B, C, and D. Access latency by the cores A, B, C, and D is shortest with the L1 cache memories L1a, L1b, L1c, and L1d, followed by the L2 cache memories L2a, L2b, L2c, and L2d, and longest with the L3 cache memory L3n. Meanwhile, the access latency with the L1, L2, and L3 cache memories is significantly shorter than with the memory module (main memory) 102. With such structures, the plurality of cores A, B, C, and D in the processor module 104 can process tasks in parallel. For example, each of the plurality of cores A, B, C, and D can perform analysis processing through comparison with a reference character string based on respective given query character strings. While a case where the cache memories are divided in three stages has been exemplified, the present embodiment is not limited thereto and the cache memories may be in one, two, or four or more stages. In the following description, the first cache memories (L1 cache memories) L1a, L1b, L1c, and L1d, the second cache memories (L2 cache memories) L2a, L2b, L2c, and L2d, and the third cache memory (L3 cache memory) L3n may be referred to collectively as "cache memory." In the present invention, the number of cores in the processor module 104 is not particularly limited, either. The number of cores can be selected in various ways, from several to several tens of thousands.

Moreover, while the configurations shown in Figs. 17 and 18 demonstrate general-purpose hardware structures, the present invention is not limited thereto. All or part of these hardware structures may employ hardware individually customized (programmed) using FPGAs or the like. In the following description, the memory module 102, the internal storage device 108, the external storage device 118, the first cache memories (L1 cache memories) L1a, L1b, L1c, and L1d, the second cache memories (L2 cache memories) L2a, L2b, L2c, and L2d, and the third cache memory (L3 cache memory) L3n, which can serve as hardware capable of storing data, may be referred to correctively as "storage devices."

Return to Fig. 1. In the present disclosure, the lower-level computers 20 process tasks in parallel under the control of the upper-level computer 10. For example, each of the plurality of lower-level computers 20 performs analysis processing through comparison with the reference character string based on respective given query character strings. In the following description, the lower-level computers 20(1) to 20(n) may be referred to simply as "lower-level computers 20" unless distinction is needed in particular.

The database 30 stores various types of data, such as the reference character string, under the control of the upper-level computer 10. For example, the database 30 stores a human genome reference sequence. The human genome reference sequence refers to data indicating a sequence of base pairs defined as a standard human genomic sequence. The database 30 also stores an index generated based on the reference character string. The index is a specific type of data array structure used to traverse for the reference character string. In the diagram, the database 30 is configured to be connected to only the upper-level computer 10 in an accessible manner. However, this is not restrictive. The lower-level computers 20 may also be configured to be connected in an accessible manner. Alternatively, the lower-level computers 20 may be equipped with similar databases. While the following description deals with a case using the human genome reference sequence, a metagenomic reference sequence combining a plurality of organisms (animals), plants, and the like may also be used.

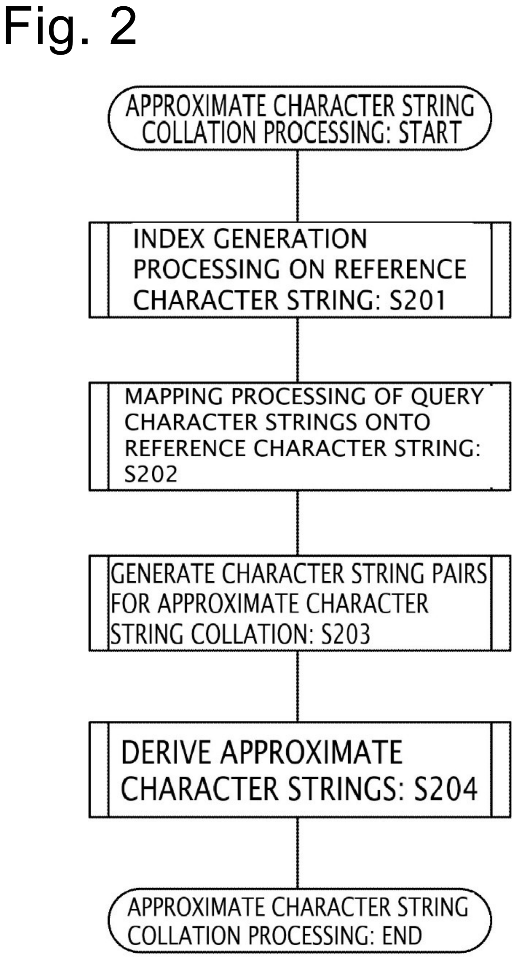

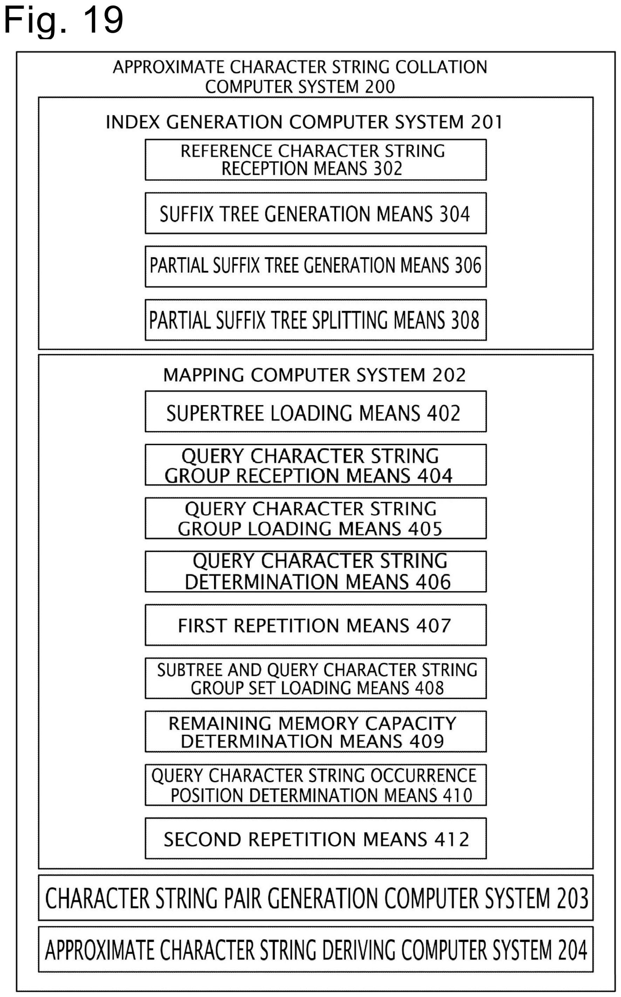

Fig. 2 is a flowchart for describing an example of an outline of approximate character string collation processing by the computer system 1 according to one embodiment of the present invention. Such processing is implemented, for example, by the upper-level computer 10 and the plurality of lower-level computer 20 running an approximate character string collation program on their processor modules 102 and thereby cooperating with other hardware components. The computer system 1, or the upper-level computer 10, or the plurality of lower-level computers 20 thereby function as an approximate character string collation computer system 200 shown in Fig. 19. The approximate character string collation program includes a plurality of processing blocks (subroutines). As a result, more specifically, the approximate character string collation computer system 200 includes, thereinside, an index generation computer system 201, a mapping computer system 202, a character string pair generation computer system 203, and an approximate character string deriving computer system 204.

As shown in Fig. 2, the upper-level computer 10 initially reads the reference character string stored in the database 30, and generates an index based on the read reference character string (index generation processing: S201). Specifically, the upper-level computer 10 generates an index having a predetermined data array structure by extending and/or sorting partial character strings of the reference character string based on predetermined conditions and arranging the resultant into an array. The data array structure has a hierarchical tree structure. In the present disclosure, such an index will be referred to as a hierarchical index. The upper-level computer 10 stores the generated index based on the reference character string into the database 30. Details of the processing for generating the hierarchical index based on the reference character string will be described below. If the hierarchical index based on the reference character string has already been generated and stored in the database 30, the index generation processing S201 can be omitted. The computer system 1 thus functions as the index generation computer system 201.

The upper-level computer 10 then receives query character strings from a not-shown external device, refers to the hierarchical index based on the received query character strings, and performs mapping processing of the query character strings onto the reference character string (mapping processing: S202). In one example, the external device is a sequencer that reads the human genome. The query character strings are base pair data fragments of, e.g., approximately 150 to 200 bases output from the sequencer. The query character strings may be once stored in the database 30. The number of characters in a query character string is preferably 50 or more, more preferably 100 or more. Meanwhile, the number of characters in the query character string is preferably 1000 or less, desirably 300 or less, more desirably 200 or less. In the present disclosure, the lower-level computers 20 also refer to the hierarchical index and perform mapping based on assigned query character strings under the control of the upper-level computer 10. The computer system 1 thereby functions as the mapping computer system 202.

Mapping is a process of identifying a partial character string (matching character string) of the reference character string that matches at least a part of a query character string. In other words, the occurrence start position and length (number of characters) of a partial character string matching at least a part of the query character string in the reference character string is identified by mapping. The mapping according to the present disclosure identifies the occurrence start position of the query character string in the reference character string by searching or traversing the hierarchical index for the query character string. For faster processing, the generated hierarchical index is preferably loaded into high-speed memory such as a cache memory in the memory module 102 or the processor module 104.

In the foregoing example, the upper-level computer 10 and the lower-level computers 20 each perform mapping based on assigned query character strings. However, this is not restrictive. For example, only the upper-level computer 10 may refer to and traverse the hierarchical index based on assigned query character strings.

Next, the upper-level computer 10 and the lower-level computers 20 each generate character string pairs for approximate character string matching to be described below (character string pair generation processing: S203). A character string pair consists of a to-be-collated character string in the reference character string and a collation character string in the query character string, which include a matching character string identified by mapping. The computer system 1 thereby functions as the character string pair generation computer system 203.

Specifically, the upper-level computer 10 and the lower-level computers 20 generate to-be-collated character strings by adding predetermined lengths of corresponding character strings in the reference character string to both the beginning and end of the matching character strings identified by mapping. In other words, the to-be-collated character strings are character strings that lie in the vicinities of the matching character strings identified in the reference character string and include the matching character strings. For example, if the reference character string is "CCGATCTGTA TACCCTACGA" and a matching character string is "TACC", a character string "TATACCCT" obtained by adding two characters each in front and behind is a to-be-collated character string. In the case of human genome analysis, the length of bases to be added to the beginning and end of the matching character strings in the reference character string can be approximately 50 bases each.

The upper-level computer 10 also generates collation character strings by adding a predetermined length of corresponding character string in the query character strings to the end of the matching character strings. In the case of human genome analysis, the length of the character strings to be added to the end of the matching character strings in the query character strings is approximately 50 bases, for example.

In the present disclosure, predetermined lengths of character strings are described to be added to the beginning and end of the matching character strings in the reference character string. However, this is not restrictive. For example, a predetermined length of character string may be added to either the beginning or the end, for example. Predetermined lengths of character strings may be added to both the beginning and end of the matching character strings in the query character strings. Alternatively, the matching character strings themselves may be handled as the collation character strings without adding a character string.

Next, the upper-level computer 10 and the lower-level computers 20 derive at least one approximate character string based on the character string pairs consisting of the to-be-collated character strings based on the reference character string and the collation character strings based on the query character strings (approximate character string deriving processing: S204). To derive an approximate character string, a predetermined alignment using dynamic programming is applied. Alignment is a technique that compares two sequences (character strings) while allowing substitution, insertion, and omission between their elements, and calculates the degree of variation (degree of similarity) based on defined scores/penalties. Known algorithms for implementing alignment include the Smith-Waterman algorithm and the Needleman-Wunsch algorithm. Dynamic programming is a technique that calculates an optimal solution for the next stage based on the optimal solution obtained at a certain stage. More specifically, in calculating the degree of variation, a score is calculated for each element of the array-like alignment table based on dynamic programming, the element with the maximum score is determined, and at least one or more approximate character strings are thereby derived. The computer system 1 thus functions as the approximate character string deriving computer system 204.

As described above, in the approximate character string collation processing of the present embodiment, a hierarchical index based on the reference character string is generated. Matching character strings (and their lengths) are then identified by traversing the hierarchical index based on given query character strings, and an approximate character string or strings are derived by performing approximate character string collation on character string pairs each consisting of a to-be-collated character string and a collation character string based on the identified matching character strings. In other words, the approximate character string collation program of the present embodiment includes an index generation program based on the reference character string, a mapping program for traversing the hierarchical index based on the query character strings, a program for generating character string pairs consisting of to-be-collated character strings and collation character strings, and an alignment program for deriving an approximate character string or strings by performing approximate character string collation using the character string pairs. Since the program for generating character string pairs and the alignment program may be conventional ones, a detailed description thereof will heretofore be omitted.

<Index Generation Program>

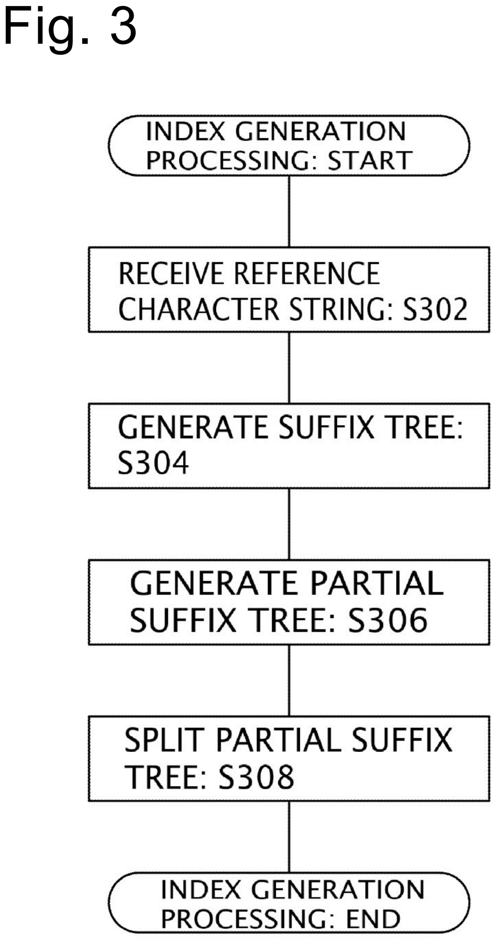

Fig. 3 is a flowchart for describing an example of the index generation processing by the computer system according to the present embodiment. In other words, Fig. 3 shows details of the hierarchical index generation processing (S201) shown in Fig. 2. Fig. 19 shows a functional block configuration of the index generation computer system 201, which includes reference character string reception means 302, suffix tree generation means 304, partial suffix tree generation means 306, and partial suffix tree splitting means 308. The functional blocks will now be described with reference to the flowchart etc.

(Reference Character String Reception Means 302)

As shown in Fig. 3, the upper-level computer 10 initially receives the reference character string for generating a hierarchical index (S302). For example, in the case of the human genome reference sequence, the upper-level computer 10 accesses the database 30 and reads the stored human genome reference sequence. For ease of understanding, the following description will be given on the assumption that the human genome reference sequence is a 20-character reference character string "CCGATCTGTATACCCTACGA" composed of four types of characters "A", "C", "G", and "T". Note that an actual human genome reference sequence includes 3 billion characters.

(Suffix Tree Generation Means 304)

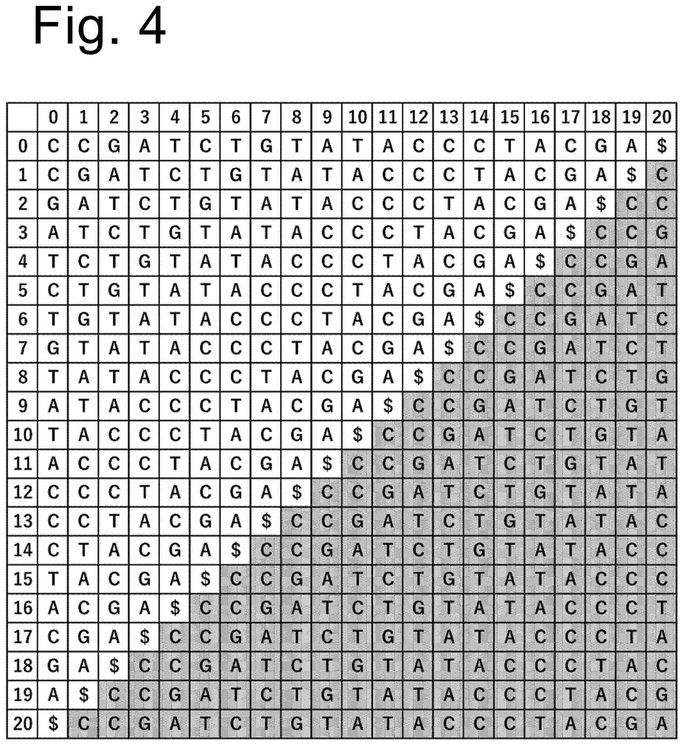

Next, the upper-level computer 10 generates a suffix tree for the received reference character string (S304). Specifically, for example, "$" is initially added as the terminal character of the reference character string. This terminal character shall be a character that is not included in the reference character string and is the smallest. The 21-character reference character string including this terminal character is cyclically shifted character by character, whereby 21 cyclic character strings are generated (see Fig. 4). In Fig. 4, the sequence numbers of the cyclic character strings are attached to the tops (left ends) of the respective cyclic character strings. The sequence numbers represent the occurrence start positions of the respective cyclic character strings with reference to the 0th cyclic character string serving as a reference (reference character string). If an actual human genome reference sequence has 3 billion characters, the number of cyclic character strings (number of sequences) is 3 billion.

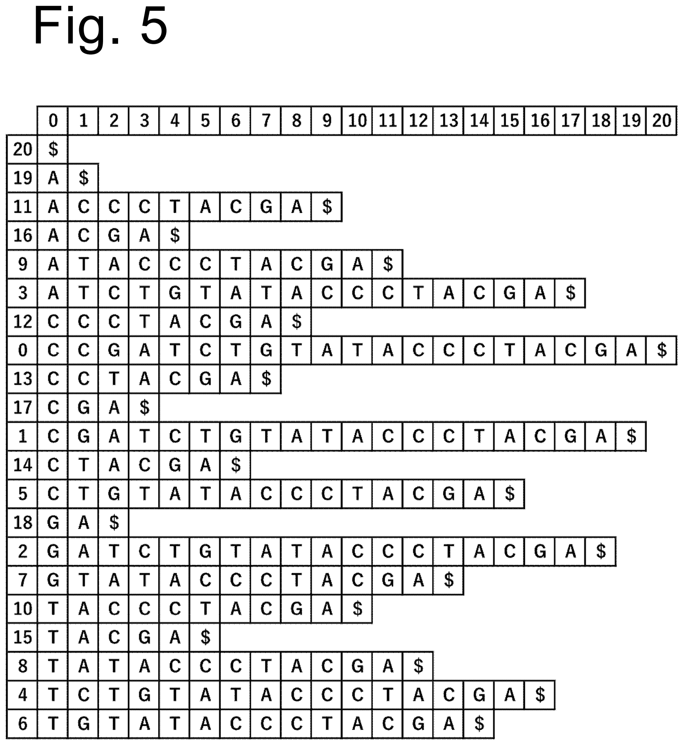

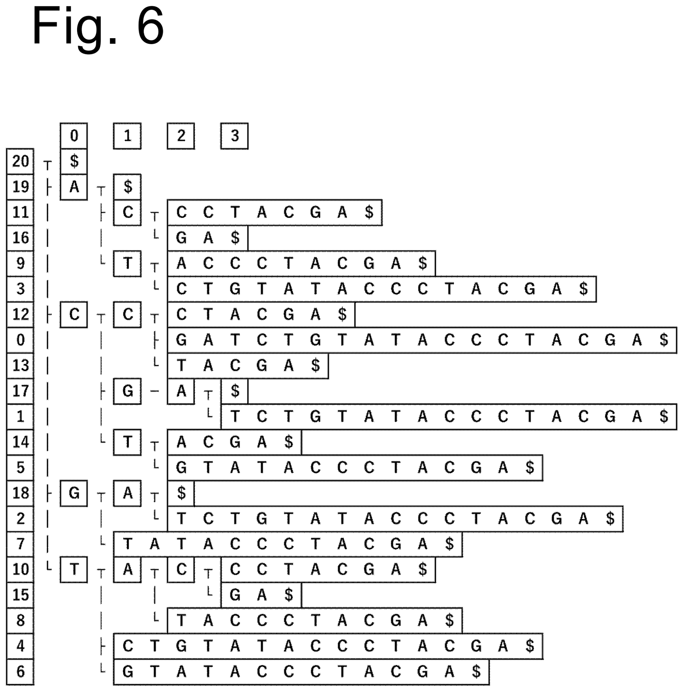

The upper-level computer 10 cuts off the character strings after the terminal character "$" in the cyclic character strings (see the shaded areas in Fig. 4) and sorts the resulting group of cyclic character strings (see Fig. 5) to generate a suffix tree expressed by a hierarchical tree structure. Fig. 6 shows this suffix tree. The number of levels of the suffix tree is shown at the top of Fig. 6. In the present embodiment, character strings remaining at the stage when the number of nodes on the lower-level side becomes 1 are grouped together at that terminal node (leaf). As a result, the number of levels of the suffix tree in the present embodiment is three, with the terminal nodes (leaves) included. In the case of an actual human genome reference sequence, the number of levels is far greater (for example, around 20 levels). The foregoing procedure for generating the suffix tree is an example for reference, and the present invention is not limited thereto. Various known techniques can be employed for the program that generates the suffix tree.

(Partial Suffix Tree Generation Means 306)

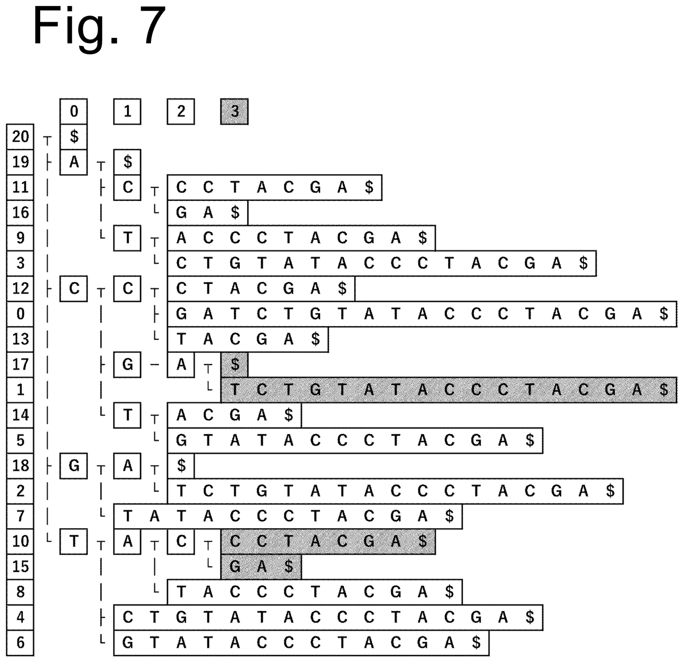

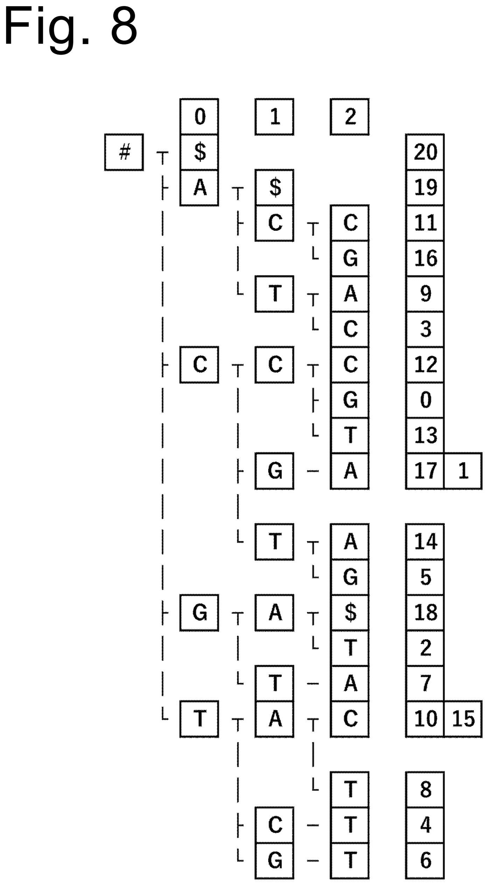

Next, the upper-level computer 10 generates a partial suffix tree from the generated suffix tree (S306). Specifically, as shown by the shaded areas in Fig. 7, hierarchical data at levels lower than a predetermined number of levels set in advance are forcefully cut off. Here, the suffix tree from which the lower-level data is cut off will be referred to as a partial suffix tree. The predetermined number of levels will be referred to as a cut level number. In the present embodiment, for the convenience of description, the cut level number is assumed to be 2, and the hierarchical data on the lower-level side is cut off. Fig. 8 shows the partial suffix tree. In the partial suffix tree of Fig. 8, if multiple characters are stored in a terminal node (leaf), the first character (or a predetermined number of characters from the top) is left and the subsequent character string is cut off to simplify the tree structure. While the suffix tree needs information about the positions and lengths of the matching cyclic character strings on respective tree nodes, the truncation of the levels enables substitution of the information with characters themselves without significantly increasing data volume. This enables faster search.

In the partial suffix tree of Fig. 8, the sequence numbers of the respective cyclic character strings are attached to the right ends of the terminal nodes (leaves) of the partial suffix tree. Take, for example, the root (#)-C-G-A system. In the original suffix tree (see Fig. 7), there are two nodes (branches) "root(#)-C-G-A-$" and "root(#)-C-G-AT..." on the lower-level side. The sequence number, 17, of the node on the higher-level side (elder-sibling side) in the sort order and the sequence number, 1, of the node on the lower-level side (younger-sibling side) are attached in this order to the right of the leaf, root(#)-C-G-A, of the partial suffix tree. Take, for example, the root(#)-T-A-C system. In the original suffix tree (see Fig. 7), there are two nodes (branches) "root(#)-T-A-C-C..." and "root(#)-T-A-C-G..." on the lower-level side. The sequence number, 10, of the node on the higher-level side (elder-sibling side) in the sort order and the sequence number, 15, of the node on the lower-level side (younger-sibling side) are therefore attached in this order to the right side of the leaf, root(#)-T-A-C, of the partial suffix tree. In such cases, it means that the occurrence position of the identified query character string is identified to fall on the two positions.

In the case of the actual huma genome reference sequence, the number of levels of the (entire) suffix tree is enormous. In such a case, the value of the cut level number to be set to generate a partial suffix tree is preferably 1000 or less, desirably 500 or less, more preferably 300 or less, yet more preferably 200 or less. On the other hand, the value of the cut level number is preferably 30 or more, desirably 50 or more, more preferably 80 or more. The value of the cut level number to be set is desirably equal to or greater than the number of characters of the query character string. For example, if the query character string is composed of 150 base pairs, the cut level number is set to 150 or greater. Specifically, if the cut level number of the human genome reference sequence is set to 150, the (entire) suffix tree (with a data volume of approximately 250 GB) is reduced to a 150-level partial suffix tree (with a data volume of approximately 100 GB). As a result, the data volume can be significantly reduced.

In the simplified example of Fig. 8, multiple sequence numbers are attached to the two systems "root(#)-C-G-A" and "root(#)-T-A-C" among the 19 terminal nodes (leaves) of the partial suffix tree. In the case of the partial suffix tree generated based on the human genome reference sequence, an enormous number of levels are reduced to 150 levels. As a result, multiple sequence numbers are attached to terminal nodes (leaves), but rarely exceed three in number. In genome searches, since mutations are actually highly likely to be present, narrowing down to a single sequence number tends to increase matches at incorrect locations.

To improve the mapping accuracy, the tree is desirably cut at a stage where multiple sequence numbers remain.

The foregoing embodiment also deals with the case where the partial suffix tree is generated by the intermediary of two means that are the suffix tree generation means 304 and the partial suffix tree generation means 306. However, the generation procedure of the partial suffix tree is not limited thereto. Any process that generates a partial suffix tree using the received reference character string can be employed.

(Partial Suffix Tree Splitting Means 308)

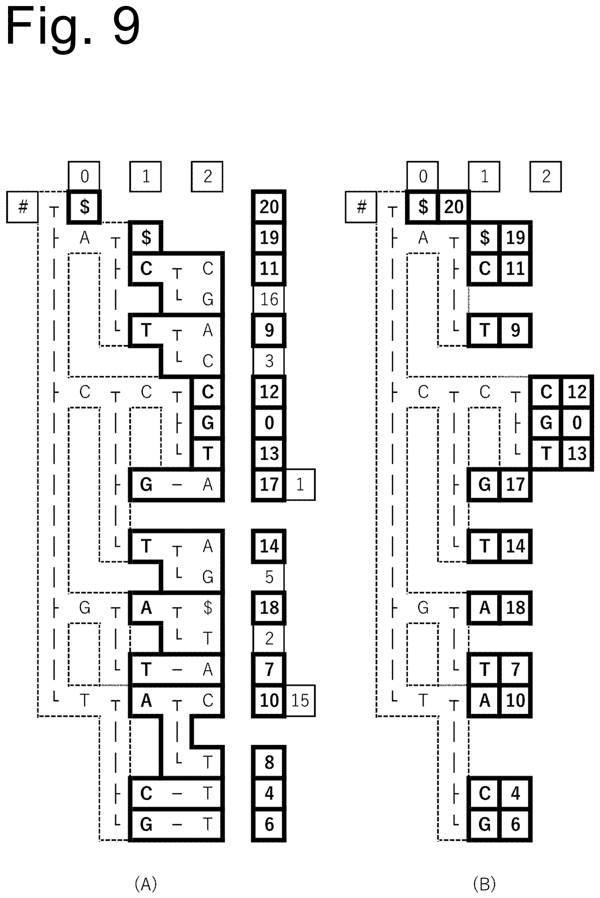

Next, the upper-level computer 10 generates a supertree including higher levels and a plurality of subtrees (hereinafter, referred to as a subtree group) connected below the supertree by splitting the partial suffix tree at an intermediate level (intermediate node) (S308). An example of the splitting condition for intermediate nodes where splitting occurs is, for example, that "when tracing back intermediate nodes from terminal nodes to higher levels, the number of terminal nodes (leaves) belonging to levels lower than that intermediate node falls to or below a predetermined value." For example, Fig. 9(A) shows a case where the partial suffix tree is split under the condition that "when tracing back intermediate nodes from terminal nodes to higher levels, the number of terminal nodes (leaves) belonging to levels lower than that intermediate node falls to or below a binary value." Here, the partial suffix tree is split between one supertree including the root (#) and 14 subtrees connected below the supertree. Each subtree is assigned data serving as a management number. The management numbers may be assigned by any rule. For example, the eldest sibling number (highest number in the sort order) among sequence numbers belonging to the subtree may be employed. Specifically, in terms of the sort order, the subtree group is generated to include: a first subtree with management number 20, split at the node "root(#)-$"; a second subtree with management number 19, split at the node "root(#)-A-$"; a third subtree with management number 11, split at the node "root(#)-A-C"; a fourth subtree with management number 9, split at the node "root(#)-A-T"; a fifth subtree with management number 12, split at the node "root(#)-C-C-C"; a sixth subtree with management number 0, split at the node "root(#)-C-C-G"; a seventh subtree with management number 13, split at the node "root(#)-C-C-T"; an eighth subtree with management number 17, split at the node "root(#)-C-G"; a ninth subtree with management number 14, split at the node "root(#)-C-T"; a tenth subtree with management number 18, split at the node "root(#)-G-A-$"; an eleventh subtree with management number 2, split at the node "root(#)-G-A-T"; a tenth subtree with management number 18, split at the node "root(#)-G-A-$"; an eleventh subtree with management number 7, split at the node "root(#)-G-T"; a twelfth subtree with management number 10, split at the node "root(#)-T-A"; a thirteenth subtree with management number 4, split at the node "root(#)-T-C"; and a fourteenth subtree with management number 6, split at the node "root(#)-T-G". The data volumes of the data fragments of the respective subtrees in the subtree group are preferably set to sizes that can be contained within the cache memories of the processor module 104 (L1 cache memories in particular). In other words, the "number (predetermined value) of terminal nodes (leaves) belonging to lower levels" in the splitting condition for intermediate nodes where splitting occurs is desirably set in consideration of the capacity of the cache memories.

Fig. 9(B) shows the partial suffix tree with the supertree, the splitting nodes (terminal nodes of the supertree), and the subtree management numbers left. As can be seen, the data volume of the data fragment of the supertree is significantly reduced by the separation from the subtrees. This supertree preferably has data volume within the memory module (main memory), and more desirably a size within the cache memories. As a result, the hierarchical index data where the partial suffix tree is split between the supertree data fragment and a subtree data fragment group is completed.

(Data Structure of Hierarchical Index Data)

As shown in Fig. 9(A), the data structure of the hierarchical index data generated according to the present embodiment is a suffix tree structure obtained by hierarchically structuring the suffix array of the reference character string. In addition, the number of levels from the uppermost level to lower levels is set to a predetermined value, whereby the hierarchical data at levels lower than the predetermined value is omitted. As a result, the data volume of the hierarchical index data can be reduced. In particular, while the number of levels of the data structure in the worst case is proportional to the length of the reference character string, the present embodiment can dramatically reduce the data volume since the predetermined value is set to 1000 or less so that a partial suffix tree with 1000 or fewer levels is used. According to the shotgun sequence method currently in widespread use, the number of characters in a query character string output from the sequencer is approximately 150 to 200. Since such query character strings can be sufficiently mapped even with a partial suffix tree with 1000 or fewer levels, the data volume can be reduced and the mapping quality can be maintained in a reasonably compatible manner.

Moreover, the data structure of the hierarchical index data generated according to the present embodiment is such that the partial suffix tree is split at an intermediate level, and includes the data fragment of the supertree including levels higher than the intermediate level and the data fragments of the plurality of subtrees including levels lower than the intermediate level and connected below the supertree. The expression "split into data fragments" means a state where the computer can store the data in the memory module 102 (storage device) as respective separate pieces of data or a state where the processor module 104 can process the data as respective separate pieces of data. Since the data volume of the supertree data fragment and the data volumes of the subtree data fragments can thereby be reduced, these data fragments can be stored in the memory module 102, and the entire data fragments can further be loaded into the cache memories. This significantly increases the probability of hits during traversal processing within the cache memories when a so-called general-purpose computer is used and the processor module 104 performs parallel mapping processing to be described below. Mapping can thus be performed at high speed.

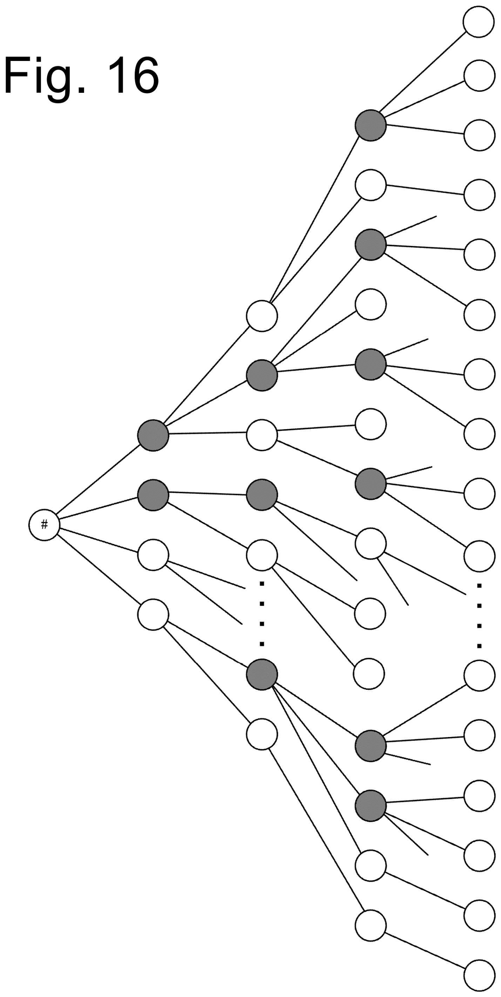

The present embodiment has dealt with the case where the number of split stages lying hierarchically midway from the terminal nodes (leaves) to the root (#) of the partial suffix tree is one. However, the present invention is not limited thereto. For example, as shown in Fig. 16, a plurality of splitting nodes (gray nodes) may be provided hierarchically midway from the terminal nodes (leaves) to the root (#) for multi-stage splitting. In Fig. 16, splitting occurs "when the number of terminal nodes (leaves) belonging to lower levels at an intermediate node on the way in tracing back from terminal nodes (leaves) to the root (#) is three or less." Moreover, in the subtree split at the splitting node, the total number of nodes is virtually converted into one, and nodes are further traced back from the splitting node to upper levels. Additional splitting occurs similarly when the number of terminal nodes (leaves) belonging to lower levels at an intermediate node on the way is three or less."

Regarding a partial suffix tree reduced to 150 levels based on the human genome reference sequence, the predetermined value in the splitting condition that "when tracking back intermediate nodes from terminal nodes to upper levels, the number of terminal nodes (leaves) belonging to levels lower than that intermediate node is less than or equal to a predetermined value" is preferably set within the range of 30,000 to 300,000. The predetermined value is desirably set to 50,000 to 200,000. For example, if the predetermined value is set to 30,000, the partial suffix tree can be split between one uppermost supertree including the root (#) and a group of approximately one million subtrees belonging to the uppermost supertree. For example, if the predetermined value is set to 100,000, the partial suffix tree can be split between one uppermost supertree including the root (#) and a group of approximately 300,000 subtrees belonging to this uppermost supertree. For example, if the predetermined value is set to 30,000, the partial suffix tree can be split between one uppermost supertree including the root (#) and a group of approximately 100,000 subtrees belonging to the uppermost supertree. Under such conditions, the uppermost supertree has a data size of 10 MB or less (for example, around 1 MB), a size at which the data can be fully loaded into the memory module 102 (main memory). Moreover, under such conditions, each subtree has a data volume of 10 MB or less, desirably 1 MB or less, a size at which the data can be fully loaded into the memory module 102 (main memory) and also fits within the cache memories.

<Mapping Program>

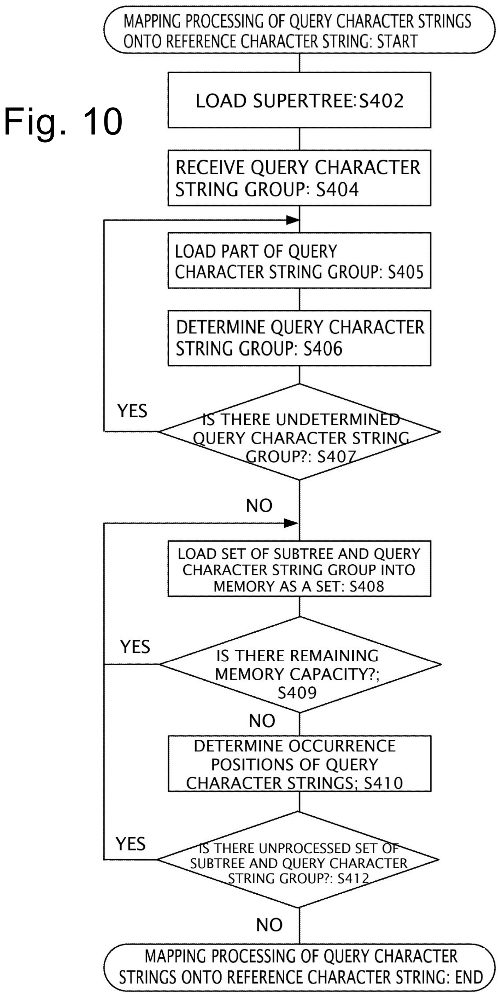

Fig. 10 is a flowchart for describing an example of the mapping processing by the computer system according to the present embodiment. In other words, Fig. 10 shows details of the mapping processing (S202) of query character strings onto the reference character string, shown in Fig. 2. By such mapping processing, the query character strings are mapped onto the reference character string, and the occurrence start positions in the reference character string are identified. Fig. 19 shows a functional block configuration of the mapping computer system 202, which includes supertree loading means 402, query character string group reception means 404, query character string group loading means 405, query character string group determination means 406, first repetition means 407, subtree and query character string group set loading means 408, remaining memory capacity determination means 409, query character string occurrence position determination means 410, and second repetition means 412. Each functional block will be described below with reference to the flowchart and the like. While the mapping processing by the upper-level computer 10 is described below, the mapping processing by the lower-level computers 20 operating in parallel is performed in a similar manner.

(Supertree Loading Means 402)

As shown in Fig. 10, the upper-level computer 10 reads the supertree of the hierarchical index from the database 30, and loads and stores the supertree into the memory module 102 (S402). In view of speedup, the upper-level computer 10 consecutively loads and stores the pieces of data constituting the supertree into the memory module 102. As employed herein, to consecutively load includes deploying the pieces of data at consecutive memory addresses on the same bank.

(Query Character String Group Reception Means 404)

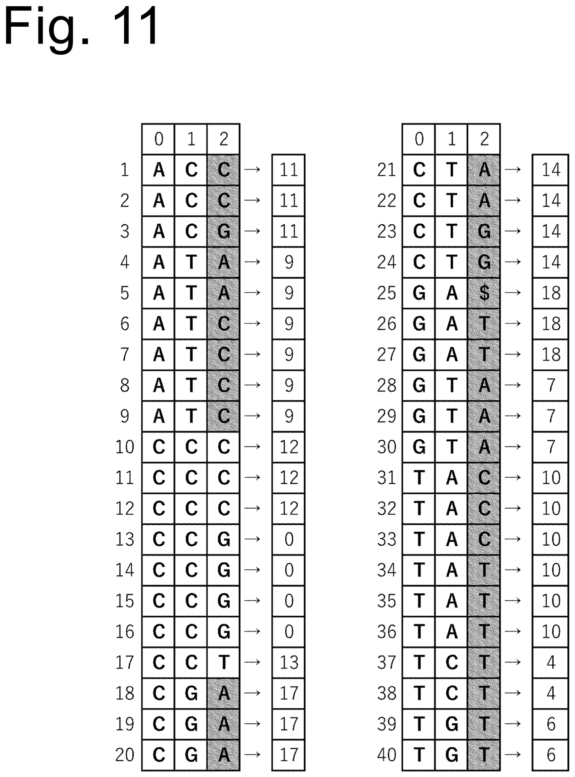

Next, the upper-level computer 10 receives a plurality of query character strings (query character string group) to be mapped onto the reference character string (S404). The received query character strings are temporarily stored in the database 30 or other storage means, for example. If, for example, the reference character string is the human genome reference sequence, the query character strings are data fragments of base pairs read from the sequencer. For ease of understanding, the following description deals with a case where there are 40 query character strings with three characters each, as shown in Fig. 11. Note that the data fragments of base pairs read from an actual sequencer are approximately 700 million query character strings with approximately 150 characters each. Although the query character string group here is described to be temporarily stored in the database 30, if the sequencer itself which is an external device has a database function, the database of the sequencer may be utilized.

(Query Character String Group Loading Means 405)

Next, the upper-level computer 10 reads the query character string group stored in the database 30, and loads and stores the query character string group into the memory module 102 (S405). In view of speedup, the upper-level computer 10 consecutively loads and stores the pieces of data constituting the query character string group into the memory module 102. Note that the entire data volume of the query character string group is enormous, and higher priority needs to be given to the loading of the supertree into the memory module 102 by the supertree loading means 402. The query character string group is thus loaded in part into the memory module 102 of the upper-level computer 10.

(Query Character String Group Determination Means 406)

Next, the upper-level computer 10 retrieves the query character strings one by one from the query character string group loaded in the memory module 102, and determines which subtree (subtree management number) the query character string belongs to by referring to the supertree loaded in the same memory module 102 (Fig. 9(B)) (S406). The subtree management numbers linked with the respective query character strings are thereby determined, and the data is stored in the memory module 102. Consequently, as shown in Fig. 11, the 40 query character strings are stored in the memory module 102 with respective belonging subtree management numbers assigned thereto. The subtree management number data linked with each query character string in the query character string group is finally stored into the database 30. As shown in Fig. 18, if the processor module includes four cores A, B, C, and D, the foregoing determination processing is preferably performed in parallel for the sake of speedup.

(First Repetition Means 407)

As described in conjunction with the query character string group loading means 405, the memory module 102 of the upper-level computer 10 can only load part of the query character string group due to its capacity upper limit. The upper-level computer 10 thus refers to the database 30 and determines whether there remains a query character string group not determined in step S406 (S407). If there is an undetermined query character string group in the database 30, the processing returns to step S405. The upper-level computer 10 loads and stores the remaining query character string group into the memory module 102, and determines the subtree management numbers linked with respective query character strings (S406).
On the other hand, if, in step S407, there no longer remains an undetermined query character string group, the processing proceeds to step S408 since all the query character strings are grouped in units of subtrees. Among the query character strings shown in Fig. 11, the shaded character strings represent ones not used during the determination referring to the supertree.

(Subtree and Query Character String Group Set Loading Means 408)

Next, the upper-level computer 10 reads a specific subtree in the hierarchical index from the database 30, and loads the subtree into the main memory module 102. The upper-level computer 10 further loads and stores a query character string group belonging to the same subtree management number as that of the loaded specific subtree into the same memory module 102. The specific subtree and the query character string group belonging thereto are thereby loaded as a set in the memory module 102 (S408). There are various methods for loading a query character string group into the memory module 102. For example, the query character string group may be arranged in an array by connecting each previous and subsequent query character strings with pointers so that the query character strings in the query character string group are consecutively read by the processor module 104.

(Remaining Memory Capacity Determination Means 409)

The upper-level computer 10 also refers to the remaining capacity of the memory module 102. If there remains free space capable of loading another specific subtree and a query character string group belonging to the same subtree management number as that of the subtree, the processing returns to step S408 to load another specific subtree and the query character string group as a set. A plurality of sets of subtrees and query character string groups are thereby concurrently loaded in the memory module 102. In the present embodiment, four sets of subtrees and query character string groups with subtree management numbers 11, 9, 12, and 0 are loaded in the memory module 102.

(Query Character String Occurrence Position Determination Means 410)

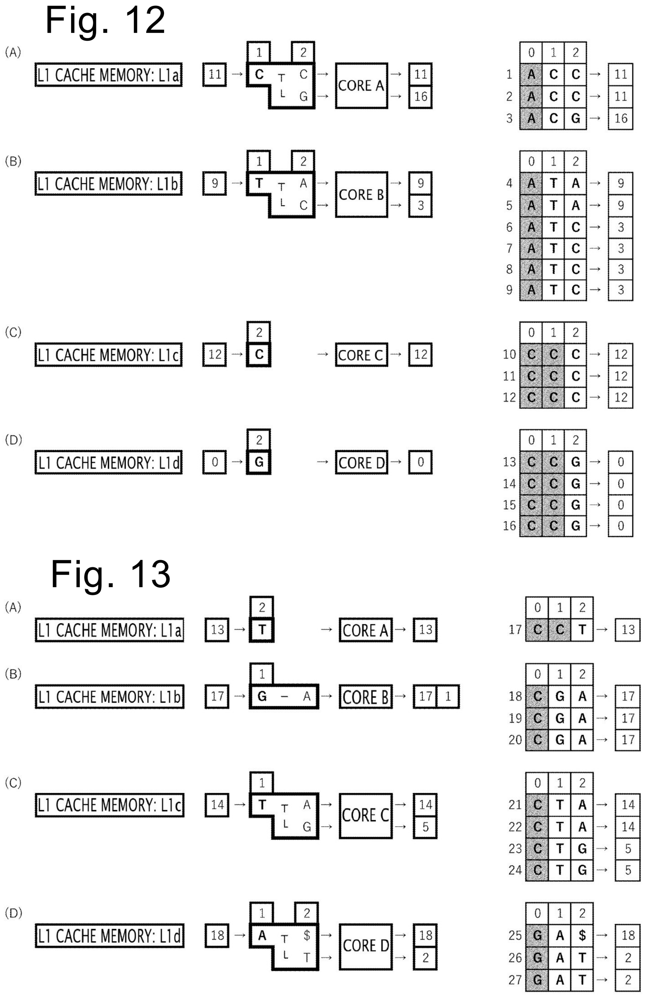

Next, the upper-level computer 10 retrieves the query character strings in the query character string groups loaded as sets in order, traverses the belonging subtrees, and determines which sequence number (occurrence position) each query character string finally belongs to (S410).
More specifically, for example, if the processor module includes four cores A, B, C, and D, parallel processing is performed by assigning the identification processing of the four sets of subtrees and query character string groups with subtree management numbers 11, 9, 12, and 0 to the cores A, B, C, and D, respectively. The data volumes of the respective subtrees are set to equal to or below the capacity of the cache memories in advance. As shown in Figs. 12(A) to 12(D), in the identification processing, the respective pieces of subtree data are loaded into the four L1 cache memories L1a, L1b, L1c, and L1d. As a result, the traversal processing of the subtrees by the respective cores A, B, C, and D is completed within the L1 cache memories L1a, L1b, L1c, and L1d, whereby high-speed processing is implemented. In Figs. 12(A) to 12(D), for the convenience of description, the numbers of levels are shown on top of top of each subtree. The management numbers of the subtrees are attached to the left of the respective subtrees. The sequence numbers (occurrence positions) of the respective terminal nodes (leaves) of each subtree are shown to the left of the subtree. The final sequence numbers (occurrence positions) are shown on the right of the respective query character strings identified by the cores A, B, C, and D. The shaded characters in the query character strings represent ones that are not used during the determination of the occurrence positions with reference to the subtrees (characters used in determining the supertree). In this step S410, the occurrence positions of all the subtrees and query character string groups loaded in the memory module 102 as sets are identified. The data about the identified results is stored into the database 30.

(Second Repetition Means 412)

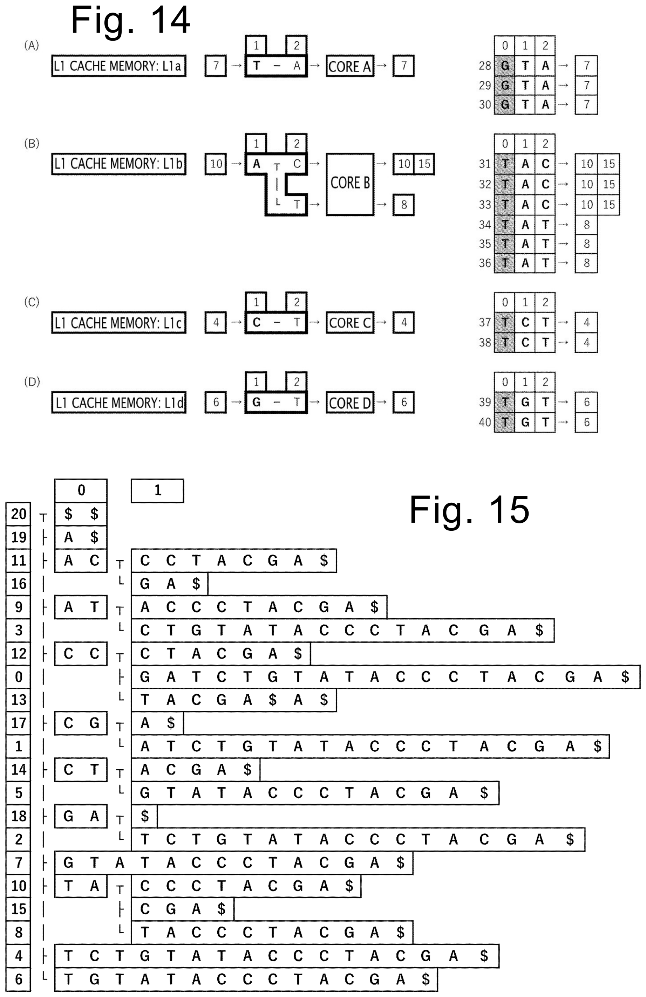

Next, the upper-level computer 10 determines whether there remains an unprocessed set of a subtree and a query character string group in the database 30 (S412). Fig. 12 shows a state where four sets of subtrees and query character strings out of the 16 sets are processed. The processing thus returns to step S408. As shown in Figs. 13(A) to 13(D), the upper-level computer 10 loads and stores the four sets of subtrees and query character strings with subtree management numbers 13, 17, 14, and 18 into the memory modules 102. The four cores A, B, C, and D then identify which sequence numbers (occurrence positions) the respective query character strings finally belong to by referring to the belonging subtrees for the query character strings in units of sets (S410). The upper-level computer 10 also determines whether there remains an unprocessed set of a subtree and a query character string group (S412). Since there still are four unprocessed sets of subtrees and query character strings, the processing returns to step S408. As shown in Figs. 14(A) to 14(D), the upper-level computer 10 loads and stores the four sets of subtrees and query character strings with subtree management numbers 7, 10, 4, and 6 into the memory modules 102. The four cores A, B, C, and D identify which sequence number (occurrence position) each query character string finally belongs to by referring to the belonging subtrees for the query character strings in units of sets (S410). Next, the upper-level computer 10 determines whether there remains any unprocessed set of a subtree and a query character string (S412). Since there is no longer any unprocessed set, the mapping processing is terminated.

Since the processing of S408 to S412 is performed by the four cores A, B, C, and D of the processor module 104 of the upper-level computer 10 in parallel, the processing time can be significantly reduced. Although not shown in particular, the plurality of lower-level computers 20(1) to 20(n)) can also perform this mapping processing simultaneously (distributed processing) for a further reduction in time.

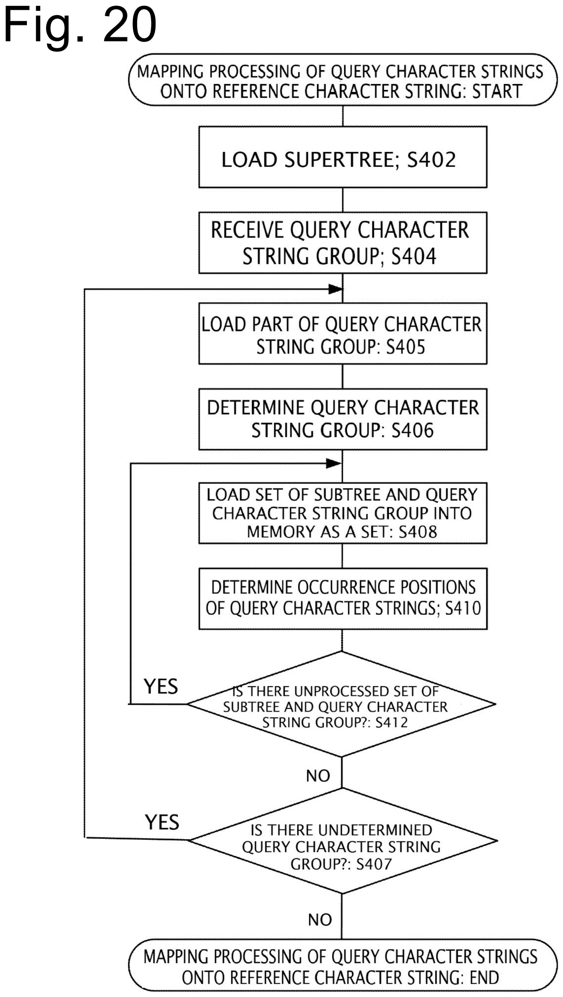

In the mapping processing of Fig. 10, if, in step S407, there remains any unprocessed query character string group, the upper-level computer 10 once writes the information related to the processed query character string groups into the database 30 to free the memory module 102, and then reads the unprocessed query character string groups from the database 30 into the memory module 102 and determines the subtree management numbers linked with the respective query character strings (S406). The processing is then described to proceed to steps S408 to S412 for identification processing after the determination processing of all the query character string groups in the database 30 is completed via step S407. However, the present invention is not limited thereto. For example, as shown in Fig. 20, when, in step S406, the determination processing is completed on the query character string groups loaded into the memory module 102 in step S405, the processing may simply proceed to step S408. Here, all the subtrees corresponding to the subtree management numbers determined by the determination processing can be retrieved and loaded as sets, followed by the identification processing in steps 410 and 412. The presence or absence of unprocessed query character strings then may be determined in step S407, and if there is any unprocessed query character string, the processing may return to step S405.

As described above, the mapping computer program according to the present embodiment can implement high-speed processing since the upper-level computer 10 determines in advance which subtrees a plurality of query character strings belong to, using the data fragment of the supertree with reduced data volume. After the completion of the determination, the subtrees and the query character string groups belonging thereto are loaded as sets, and the cores A, B, C, and D of the processor module 104 successively traverse for the plurality of query character strings belonging to the specific subtrees, whereby the occurrence positions of the query character strings can be successively identified. In the identification processing, the number of random accesses to the memory module 102 can be significantly reduced since the subtrees are taken into the cache memories.

The foregoing mapping processing demonstrates a case where a reference character string matching the entire query character string is identified. However, the present invention is not limited thereto. For example, a partial character string may be extracted from the query character string, and the mapping processing may be performed on this partial character string.

<Modification of Data Structure of Hierarchical Index>

The suffix tree employed for the hierarchical index according to the foregoing embodiment demonstrates a case where the suffix tree is generated, as shown in Fig. 6, through character-by-character hierarchical structuring (tree structuring) of a plurality of cyclic character strings (see Fig. 5) that are obtained by cyclically shifting the ACGT reference character string character by character. However, the present invention is limited thereto. The hierarchical suffix tree may be generated with a plurality of characters in the cyclic character strings (see Fig. 5) as a set. Fig. 15 shows a case where character string pairs are formed from the cyclic character strings (see Fig. 5) in units of two characters, and a suffix tree is generated from the character string pairs. This reduces the number of levels, whereby the data volumes of the partial suffix, the supertree, the subtrees, and the like can be further reduced. On the other hand, hierarchical structuring with five or more characters as a set, for example, may increase the number of nodes and can rather result in an increase in data volume. When the suffix tree is generated with a plurality of character strings as a set, the number of characters in the set is therefore preferably two or more and four or less.

The foregoing embodiments are examples for describing the present invention, and not intended to limit the present invention solely to these embodiments. The present invention can be practiced in various forms without departing from the gist thereof. For example, the present embodiment has dealt with the case where the hierarchical index is generated and mapping is performed for the reference character string that is genetic information. However, the present invention is not limited thereto, and can be applied to a case where a hierarchical index is generated and mapping is performed for a reference character string other than genetic information.

Moreover, for example, the steps, operations, or functions of the methods disclosed in this description may be performed in parallel or in different order, unless inconsistency arises in the results. The described steps, operations, and functions are provided merely as examples. Some of the steps, operations, and functions may be omitted or mutually combined into one without departing from the gist of the invention. Other steps, operations, or functions may be added.

The foregoing embodiment has also dealt with the case where the hardware configuration of the computing devices has a general-purpose structure. However, the present invention is not limited thereto. For example, individually customized pieces of hardware using an FPGA (Field Programmable Gate Array) and the like may be employed. Specifically, all or part of the functional blocks illustrated in Fig. 19 can be implemented using an FPGA.

Specific representation methods (notation methods) for implementing the data structure of the hierarchical index described in the foregoing embodiment are not limited in particular. For example, the tree structure can be represented using the LOUDS (Level-Ordered Unary Degree Sequence) technique for a further reduction in the amount of memory.

Furthermore, although various embodiments are disclosed in this specification, specific features (technical matters) in one embodiment can be added to other embodiments, or replaced with specific features in those other embodiments, while appropriately modifying them as needed, and such configurations are also included in the scope of the present invention.

## Claims

1.  An index generation computer program for causing a computing device to generate an index to be used in identifying a partial character string of a reference character string based on a query character string, the index generation computer program comprising the step of:
    setting a number of levels from an uppermost level to a lower level in a suffix tree, which is obtained by hierarchically structuring a suffix array of the reference character string, to a predetermined value and thereby omitting hierarchical data at levels lower than the predetermined value.

2.  The index generation computer program according to claim 1, wherein the predetermined value of the number of levels is set to 1000 or less.

3.  The index generation computer program according to claim 1, wherein the predetermined value of the number of levels is set to be greater than or equal to a number of characters in the query character string.

4.  The index generation computer program according to claim 1, further comprising the step of generating a supertree including a level higher than an intermediate level and a plurality of subtrees (hereinafter, referred to as a subtree group) including a level lower than the intermediate level and connected below the supertree by splitting the suffix tree at the intermediate level.

5.  The index generation computer program according to claim 4, wherein data volume of the subtrees is set to a value capable of storage in a cache memory of the computing device.

6.  The index generation computer program according to claim 4, wherein data volume of the subtrees is set to 10 MB or less.

7. An index generation computer program for causing a computing device to generate an index to be used in identifying a partial character string of a reference character string based on a query character string, the index generation computer program comprising the step of

generating a supertree including a level higher than an intermediate level and a plurality of subtrees (hereinafter, referred to as a subtree group) including a level lower than the intermediate level and connected below the supertree by splitting a suffix tree, which is obtained by hierarchically structuring a suffix array of the reference character string, at the intermediate level.

8. A mapping computer program for causing a computing device to implement a method for identifying a partial character string of a reference character string based on a query character string, wherein:

the mapping computer program comprises the index generation computer program according to any one of claims 1 to 7 as a part; and

the method further includes referring to the hierarchical index and mapping the query character string onto the reference character string to identify a partial character string of the reference character string that matches at least a part of the query character string.

9. A mapping computer program for causing a computing device to implement a method for identifying a partial character string of a reference character string based on a query character string, wherein:

a hierarchical index generated based on the reference character string is split at an intermediate level and thereby includes a supertree including a level higher than the intermediate level and a plurality of subtrees (hereinafter, referred to as a subtree group) including a level lower than the intermediate level and connected below the supertree;

the method comprises referring to the hierarchical index and mapping the query character string onto the reference character string to identify a partial character string of the reference character string that matches at least a part of the query character string; and

the mapping of the query character string includes

a determination step of performing collation processing on the plurality of query character strings using the supertree and thereby determining which of the subtrees in the subtree group each of the query character strings belongs to, and

an occurrence position determination step of performing collation processing using a specific one of the subtrees on the plurality of query character strings determined to belong to the specific one of the subtrees in the determination step and thereby determining occurrence positions of the query character strings relative to the reference character string.

10. The mapping computer program according to claim 9, wherein the occurrence position determination step of the subtrees is processed in parallel.

11. The mapping computer program according to claim 9, wherein the determination step is processed in parallel.

12. The mapping computer program according to claim 9, wherein the subtrees is loaded into a cache memory of the computing device in the occurrence position determination step.

13. The mapping computer program according to claim 9, wherein the index generation computer program according to any one of claims 1 to 7 is included as a part.

14. An index generation computer apparatus including a computing device including a storage device, the index generation computer apparatus being configured to generate an index to be used in identifying a partial character string of a reference character string based on a query character string, the computing device comprising:
partial suffix tree generation means for reading the reference character string stored in the storage device, setting a number of levels from an uppermost level to a lower level in a suffix tree, which is obtained by hierarchically structuring a suffix array of the reference character string, to a predetermined value, and generating a partial suffix tree where hierarchical data at levels lower than the predetermined value is omitted.

15. An index generation computer apparatus including a computing device including a storage device, the index generation computer apparatus being configured to generate an index to be used in identifying a partial character string of a reference character string based on a query character string, the computing device comprising:
suffix tree splitting means for generating a supertree including an upper level and a plurality of subtrees (hereinafter, referred to as a subtree group) connected below the supertree by splitting a suffix tree, which is obtained by hierarchically structuring a suffix array of the reference character string stored in the storage device, at an intermediate level.

16. A mapping computer apparatus including a computing device including a storage device, the mapping computer apparatus being configured to identify a partial character string of a reference character string based on a query character string, wherein:

the storage device stores a supertree including a level higher than an intermediate level and a plurality of subtrees (hereinafter, referred to as a subtree group) including a level lower than the intermediate level and connected below the supertree, the supertree and the subtree group being generated by splitting a hierarchical index, which is generated based on the reference character string, at the intermediate level;
the computing device comprises mapping means for referring to the supertree and the subtrees and mapping the query character string onto the reference character string to identify a partial character string of the reference character string that matches at least a part of the query character string; and
the mapping means includes

determination means for referring to the supertree stored in the storage device, performing collation processing on the plurality of query character strings, and thereby determining which one of the subtrees in the subtree group each of the query character strings belongs to, and
occurrence position determination means for referring to a specific one of the subtrees stored in the storage device, performing collation processing using a specific one of the subtrees on the plurality of query character strings determined to belong to the specific one of the subtrees by the determination means, and thereby determining occurrence positions of the query character strings relative to the reference character string.

17. A data structure of a hierarchical index of a reference character string to be used by a mapping computer program for causing a computing device to implement a method for identifying a partial character string of the reference character string based on a query character string, wherein
the data structure is a suffix tree obtained by hierarchically structuring a suffix array of the reference character string, a number of levels from an uppermost level to a lower level is set to a predetermined value, and hierarchical data at levels lower than the predetermined value are omitted.

18. The data structure of the hierarchical index according to claim 16, wherein the hierarchical index includes a data fragment of a supertree including a level higher than an intermediate level and data fragments of a plurality of subtrees including a level lower than the intermediate level and connected below the supertree by the suffix tree being split at the intermediate level.

19. A data structure of a hierarchical index of a reference character string to be used by a mapping computer program for causing a computing device to implement a method for identifying a partial character string of the reference character string based on a query character string, wherein
the hierarchical index is split at an intermediate level and thereby includes a data fragment of a supertree including a level higher than the intermediate level and data fragments of a plurality of subtrees including a level lower than the intermediate level and connected below the supertree.

# Fig. 1

COMPUTER SYSTEM 1

INTERFACE

UPPER-LEVEL COMPUTER 10

DATABASE 30

LOWER-LEVEL COMPUTER 20(1)

LOWER-LEVEL COMPUTER 20(2)

LOWER-LEVEL COMPUTER 20(3)

LOWER-LEVEL COMPUTER 20(3)

# Fig. 2

```
┌─────────────────────────────────────┐
│  APPROXIMATE CHARACTER STRING        │
│  COLLATION PROCESSING: START         │
└─────────────────────────────────────┘
                    │
┌─────────────────────────────────────┐
│  INDEX GENERATION                    │
│  PROCESSING ON REFERENCE             │
│  CHARACTER STRING: S201              │
└─────────────────────────────────────┘
                    │
┌─────────────────────────────────────┐
│  MAPPING PROCESSING OF QUERY         │
│  CHARACTER STRINGS ONTO              │
│  REFERENCE CHARACTER STRING:         │
│  S202                                │
└─────────────────────────────────────┘
                    │
┌─────────────────────────────────────┐
│  GENERATE CHARACTER STRING PAIRS     │
│  FOR APPROXIMATE CHARACTER           │
│  STRING COLLATION: S203              │
└─────────────────────────────────────┘
                    │
┌─────────────────────────────────────┐
│  DERIVE APPROXIMATE                  │
│  CHARACTER STRINGS: S204             │
└─────────────────────────────────────┘
                    │
┌─────────────────────────────────────┐
│  APPROXIMATE CHARACTER STRING        │
│  COLLATION PROCESSING: END           │
└─────────────────────────────────────┘
```

# Fig. 3

```
┌─────────────────────────────┐
│     INDEX GENERATION         │
│   PROCESSING: START          │
└─────────────────────────────┘
              │
┌─────────────────────────────┐
│   RECEIVE REFERENCE          │
│   CHARACTER STRING: S302     │
└─────────────────────────────┘
              │
┌─────────────────────────────┐
│   GENERATE SUFFIX TREE:      │
│   S304                       │
└─────────────────────────────┘
              │
┌─────────────────────────────┐
│   GENERATE PARTIAL           │
│   SUFFIX TREE: S306          │
└─────────────────────────────┘
              │
┌─────────────────────────────┐
│   SPLIT PARTIAL SUFFIX       │
│   TREE: S308                 │
└─────────────────────────────┘
              │
┌─────────────────────────────┐
│     INDEX GENERATION         │
│   PROCESSING: END            │
└─────────────────────────────┘
```

# Fig. 4

|    | 0 | 1 | 2 | 3 | 4 | 5 | 6 | 7 | 8 | 9 | 10 | 11 | 12 | 13 | 14 | 15 | 16 | 17 | 18 | 19 | 20 |
|----|---|---|---|---|---|---|---|---|---|---|----|----|----|----|----|----|----|----|----|----|----|
| 0  | C | C | G | A | T | C | T | G | T | A | T  | A  | C  | C  | C  | T  | A  | C  | G  | A  | $  |
| 1  | C | G | A | T | C | T | G | T | A | T | A  | C  | C  | C  | T  | A  | C  | G  | A  | $  | C  |
| 2  | G | A | T | C | T | G | T | A | T | A | C  | C  | C  | T  | A  | C  | G  | A  | $  | C  | C  |
| 3  | A | T | C | T | G | T | A | T | A | C | C  | C  | T  | A  | C  | G  | A  | $  | C  | C  | G  |
| 4  | T | C | T | G | T | A | T | A | C | C | C  | T  | A  | C  | G  | A  | $  | C  | C  | G  | A  |
| 5  | C | T | G | T | A | T | A | C | C | C | T  | A  | C  | G  | A  | $  | C  | C  | G  | A  | T  |
| 6  | T | G | T | A | T | A | C | C | C | T | A  | C  | G  | A  | $  | C  | C  | G  | A  | T  | C  |
| 7  | G | T | A | T | A | C | C | C | T | A | C  | G  | A  | $  | C  | C  | G  | A  | T  | C  | T  |
| 8  | T | A | T | A | C | C | C | T | A | C | G  | A  | $  | C  | C  | G  | A  | T  | C  | T  | G  |
| 9  | A | T | A | C | C | C | T | A | C | G | A  | $  | C  | C  | G  | A  | T  | C  | T  | G  | T  |
| 10 | T | A | C | C | C | T | A | C | G | A | $  | C  | C  | G  | A  | T  | C  | T  | G  | T  | A  |
| 11 | A | C | C | C | T | A | C | G | A | $ | C  | C  | G  | A  | T  | C  | T  | G  | T  | A  | T  |
| 12 | C | C | C | T | A | C | G | A | $ | C | C  | G  | A  | T  | C  | T  | G  | T  | A  | T  | A  |
| 13 | C | C | T | A | C | G | A | $ | C | C | G  | A  | T  | C  | T  | G  | T  | A  | T  | A  | C  |
| 14 | C | T | A | C | G | A | $ | C | C | G | A  | T  | C  | T  | G  | T  | A  | T  | A  | C  | C  |
| 15 | T | A | C | G | A | $ | C | C | G | A | T  | C  | T  | G  | T  | A  | T  | A  | C  | C  | C  |
| 16 | A | C | G | A | $ | C | C | G | A | T | C  | T  | G  | T  | A  | T  | A  | C  | C  | C  | T  |
| 17 | C | G | A | $ | C | C | G | A | T | C | T  | G  | T  | A  | T  | A  | C  | C  | C  | T  | A  |
| 18 | G | A | $ | C | C | G | A | T | C | T | G  | T  | A  | T  | A  | C  | C  | C  | T  | A  | C  |
| 19 | A | $ | C | C | G | A | T | C | T | G | T  | A  | T  | A  | C  | C  | C  | T  | A  | C  | G  |
| 20 | $ | C | C | G | A | T | C | T | G | T | A  | T  | A  | C  | C  | C  | T  | A  | C  | G  | A  |

# Fig. 5

| | 0 | 1 | 2 | 3 | 4 | 5 | 6 | 7 | 8 | 9 | 10 | 11 | 12 | 13 | 14 | 15 | 16 | 17 | 18 | 19 | 20 |
|---|---|---|---|---|---|---|---|---|---|---|---|---|---|---|---|---|---|---|---|---|---|
| 20 | $ | | | | | | | | | | | | | | | | | | | | |
| 19 | A | $ | | | | | | | | | | | | | | | | | | | |
| 11 | A | C | C | C | T | A | C | G | A | $ | | | | | | | | | | | |
| 16 | A | C | G | A | $ | | | | | | | | | | | | | | | | |
| 9 | A | T | A | C | C | C | T | A | C | G | A | $ | | | | | | | | | |
| 3 | A | T | C | T | G | T | A | T | A | C | C | C | T | A | C | G | A | $ | | | |
| 12 | C | C | C | T | A | C | G | A | $ | | | | | | | | | | | | |
| 0 | C | C | G | A | T | C | T | G | T | A | T | A | C | C | C | T | A | C | G | A | $ |
| 13 | C | C | T | A | C | G | A | $ | | | | | | | | | | | | | |
| 17 | C | G | A | $ | | | | | | | | | | | | | | | | | |
| 1 | C | G | A | T | C | T | G | T | A | T | A | C | C | C | T | A | C | G | A | $ | |
| 14 | C | T | A | C | G | A | $ | | | | | | | | | | | | | | |
| 5 | C | T | G | T | A | T | A | C | C | C | T | A | C | G | A | $ | | | | | |
| 18 | G | A | $ | | | | | | | | | | | | | | | | | | |
| 2 | G | A | T | C | T | G | T | A | T | A | C | C | C | T | A | C | G | A | $ | | |
| 7 | G | T | A | C | C | C | T | A | C | G | A | $ | | | | | | | | | |
| 10 | T | A | C | C | C | T | A | C | G | A | $ | | | | | | | | | | |
| 15 | T | A | C | G | A | $ | | | | | | | | | | | | | | | |
| 8 | T | A | T | A | C | C | C | T | A | C | G | A | $ | | | | | | | | |
| 4 | T | C | T | G | T | A | T | A | C | C | C | T | A | C | G | A | $ | | | | |
| 6 | T | G | T | A | T | A | C | C | C | T | A | C | G | A | $ | | | | | | |

# Fig. 6

EP 4 708 300 A1

# Fig. 7

23

Fig. 8

# Fig. 9

(A)                                              (B)

Fig. 10

MAPPING PROCESSING OF QUERY CHARACTER STRINGS ONTO REFERENCE CHARACTER STRING: START

LOAD SUPERTREE:S402

RECEIVE QUERY CHARACTER STRING GROUP: S404

LOAD PART OF QUERY CHARACTER STRING GROUP: S405

DETERMINE QUERY CHARACTER STRING GROUP: S406

IS THERE UNDETERMINED QUERY CHARACTER STRING GROUP?: S407

YES

NO

LOAD SET OF SUBTREE AND QUERY CHARACTER STRING GROUP INTO MEMORY AS A SET: S408

IS THERE REMAINING MEMORY CAPACITY?; S409

YES

NO

DETERMINE OCCURRENCE POSITIONS OF QUERY CHARACTER STRINGS; S410

IS THERE UNPROCESSED SET OF SUBTREE AND QUERY CHARACTER STRING GROUP?: S412

YES

NO

MAPPING PROCESSING OF QUERY CHARACTER STRINGS ONTO REFERENCE CHARACTER STRING: END

# Fig. 11

|    | 0 | 1 | 2 |  |    |
|----|---|---|---|---|----|
| 1  | A | C | C | → | 11 |
| 2  | A | C | C | → | 11 |
| 3  | A | C | G | → | 11 |
| 4  | A | T | A | → | 9  |
| 5  | A | T | A | → | 9  |
| 6  | A | T | C | → | 9  |
| 7  | A | T | C | → | 9  |
| 8  | A | T | C | → | 9  |
| 9  | A | T | C | → | 9  |
| 10 | C | C | C | → | 12 |
| 11 | C | C | C | → | 12 |
| 12 | C | C | C | → | 12 |
| 13 | C | C | G | → | 0  |
| 14 | C | C | G | → | 0  |
| 15 | C | C | G | → | 0  |
| 16 | C | C | G | → | 0  |
| 17 | C | C | T | → | 13 |
| 18 | C | G | A | → | 17 |
| 19 | C | G | A | → | 17 |
| 20 | C | G | A | → | 17 |

|    | 0 | 1 | 2 |  |    |
|----|---|---|---|---|----|
| 21 | C | T | A | → | 14 |
| 22 | C | T | A | → | 14 |
| 23 | C | T | G | → | 14 |
| 24 | C | T | G | → | 14 |
| 25 | G | A | $ | → | 18 |
| 26 | G | A | T | → | 18 |
| 27 | G | A | T | → | 18 |
| 28 | G | T | A | → | 7  |
| 29 | G | T | A | → | 7  |
| 30 | G | T | A | → | 7  |
| 31 | T | A | C | → | 10 |
| 32 | T | A | C | → | 10 |
| 33 | T | A | C | → | 10 |
| 34 | T | A | T | → | 10 |
| 35 | T | A | T | → | 10 |
| 36 | T | A | T | → | 10 |
| 37 | T | C | T | → | 4  |
| 38 | T | C | T | → | 4  |
| 39 | T | G | T | → | 6  |
| 40 | T | G | T | → | 6  |

# Fig. 12

# Fig. 13

# Fig. 14

# Fig. 15

# Fig. 16

# Fig. 17

COMPUTING DEVICE
100

PROCESSOR
MODULE 104

MEMORY
MODULE 102

CHIPSET 106

INTERNAL
STORAGE DEVICE
108

I/O CONTROLLER
110

COMMUNICATION
INTERFACE 116

INPUT/OUTPUT
INTERFACE 114

OUTPUT
INTERFACE 112

EXTERNAL
STORAGE DEVICE
118

# Fig. 18

PROCESSOR MODULE 104

| L3 CACHE L3n | L2 CACHE L2a | L1 CACHE L1a | CORE A |
| | L2 CACHE L2b | L1 CACHE L1b | CORE B |
| | L2 CACHE L2c | L1 CACHE L1c | CORE C |
| | L2 CACHE L2d | L1 CACHE L1d | CORE D |

# Fig. 19

**APPROXIMATE CHARACTER STRING COLLATION COMPUTER SYSTEM 200**

**INDEX GENERATION COMPUTER SYSTEM 201**

REFERENCE CHARACTER STRING RECEPTION MEANS 302

SUFFIX TREE GENERATION MEANS 304

PARTIAL SUFFIX TREE GENERATION MEANS 306

PARTIAL SUFFIX TREE SPLITTING MEANS 308

**MAPPING COMPUTER SYSTEM 202**

SUPERTREE LOADING MEANS 402

QUERY CHARACTER STRING GROUP RECEPTION MEANS 404

QUERY CHARACTER STRING GROUP LOADING MEANS 405

QUERY CHARACTER STRING DETERMINATION MEANS 406

FIRST REPETITION MEANS 407

SUBTREE AND QUERY CHARACTER STRING GROUP SET LOADING MEANS 408

REMAINING MEMORY CAPACITY DETERMINATION MEANS 409

QUERY CHARACTER STRING OCCURRENCE POSITION DETERMINATION MEANS 410

SECOND REPETITION MEANS 412

**CHARACTER STRING PAIR GENERATION COMPUTER SYSTEM 203**

**APPROXIMATE CHARACTER STRING DERIVING COMPUTER SYSTEM 204**

# Fig. 20

```
┌─────────────────────────────────────────┐
│  MAPPING PROCESSING OF QUERY CHARACTER   │
│  STRINGS ONTO REFERENCE CHARACTER        │
│  STRING: START                           │
└─────────────────────────────────────────┘
                    │
                    ▼
┌─────────────────────────────────────────┐
│        LOAD SUPERTREE; S402             │
└─────────────────────────────────────────┘
                    │
                    ▼
┌─────────────────────────────────────────┐
│      RECEIVE QUERY CHARACTER            │
│      STRING GROUP; S404                 │
└─────────────────────────────────────────┘
                    │
                    ▼
┌─────────────────────────────────────────┐
│    LOAD PART OF QUERY CHARACTER         │
│      STRING GROUP: S405                 │
└─────────────────────────────────────────┘
                    │
                    ▼
┌─────────────────────────────────────────┐
│    DETERMINE QUERY CHARACTER            │
│      STRING GROUP: S406                 │
└─────────────────────────────────────────┘
                    │
                    ▼
┌─────────────────────────────────────────┐
│   LOAD SET OF SUBTREE AND QUERY         │
│  CHARACTER STRING GROUP INTO            │
│   MEMORY AS A SET: S408                 │
└─────────────────────────────────────────┘
                    │
                    ▼
┌─────────────────────────────────────────┐
│   DETERMINE OCCURRENCE POSITIONS        │
│  OF QUERY CHARACTER STRINGS; S410       │
└─────────────────────────────────────────┘
                    │
                    ▼
       IS THERE UNPROCESSED SET OF
  YES  SUBTREE AND QUERY CHARACTER
  ◄─── STRING GROUP?: S412
                    │ NO
                    ▼
       IS THERE UNDETERMINED
  YES  QUERY CHARACTER STRING
  ◄─── GROUP?: S407
                    │ NO
                    ▼
┌─────────────────────────────────────────┐
│  MAPPING PROCESSING OF QUERY CHARACTER   │
│  STRINGS ONTO REFERENCE CHARACTER        │
│  STRING: END                             │
└─────────────────────────────────────────┘
```

## INTERNATIONAL SEARCH REPORT

| | International application No. |
|---|---|
| | **PCT/JP2024/018489** |

| A. | CLASSIFICATION OF SUBJECT MATTER |
|---|---|

*G16B 15/00*(2019.01)i; *G06F 16/332*(2019.01)i; *G16B 20/00*(2019.01)i
FI:   G16B15/00; G16B20/00; G06F16/332

According to International Patent Classification (IPC) or to both national classification and IPC

| B. | FIELDS SEARCHED |
|---|---|

Minimum documentation searched (classification system followed by classification symbols)

G16B15/00; G06F16/332; G16B20/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

| C. | DOCUMENTS CONSIDERED TO BE RELEVANT |
|---|---|

| Category* | Citation of document, with indication, where appropriate, of the relevant passages | Relevant to claim No. |
|---|---|---|
| A | JP 2002-229987 A (INTERNATIONAL BUSINESS MACHINES CORPORATION) 16 August 2002 (2002-08-16)<br>entire text, all drawings | 1-19 |
| A | US 2020/0265923 A1 (THE REGENTS OF THE UNIVERSITY OF MICHIGAN) 20 August 2020 (2020-08-20)<br>entire text, all drawings | 1-16 |
| X | paragraph [0012] | 17-19 |
| A | EP 3136607 A1 (INSTITUTE OF MATHEMATICS AND COMPUTER SCIENCE, UNIVERSITY OF LATVIA) 01 March 2017 (2017-03-01)<br>entire text, all drawings | 1-19 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **05 June 2024** | **18 June 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (July 2022)

**INTERNATIONAL SEARCH REPORT**
Information on patent family members

International application No.

**PCT/JP2024/018489**

| Patent document cited in search report | | | Publication date (day/month/year) | Patent family member(s) | | | Publication date (day/month/year) |
|---|---|---|---|---|---|---|---|
| JP | 2002-229987 | A | 16 August 2002 | US | 2002/0123995 | A1 | |
| US | 2020/0265923 | A1 | 20 August 2020 | (Family: none) | | | |
| EP | 3136607 | A1 | 01 March 2017 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **HENG LI**. Aligning sequence reads, clone sequences and assembly contigs with BWA-MEM. *arXiv: 1303.3997 (q-bio.GN)*, 26 May 2013